(19) Europäisches Patentamt
European Patent Office
Office européen des brevets

(11) EP 3 632 401 A1

(12) EUROPEAN PATENT APPLICATION

(43) Date of publication:
08.04.2020 Bulletin 2020/15

(51) Int Cl.:
*A61K 6/10* (2006.01)

(21) Application number: 18198048.3

(22) Date of filing: 01.10.2018

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicants:
- Dentsply Sirona Inc.
York, PA 17401 (US)
- Dentsply DeTrey GmbH
78467 Konstanz (DE)

(72) Inventors:
- Jin, Xiaoming
Middletown, DE 19709 (US)
- Angeletakis, Christos
Bear, DE 19701 (US)
- Liu, Yi
Dover, DE 19901 (US)
- Klee, Joachim E.
78315 Radolfzell (DE)
- Weber, Christoph
78464 Konstanz (DE)
- Huo, Xin
Dover, DE 19901 (US)

(74) Representative: Dietz, Mirko
Sirona Dental Systems GmbH
Fabrikstraße 31
64625 Bensheim (DE)

# (54) MACROMONOMER BASED LIGHT-CURABLE DENTAL IMPRESSION MATERIAL

(57) A light-curable dental impression material comprising
(a) a polymerizable polysiloxane resin composition comprising compounds of formula (I)

$$E\text{-}(L^1\text{-}Z)_n\text{-}L^2\text{-}E \qquad (I)$$

(b) a particulate filler; and
(c) a photoinitiator.

EP 3 632 401 A1

## Description

### Field of the Invention

The present invention relates to a light-curable dental impression material. Moreover, the present invention relates to a use of the light-curable dental impression material of the present invention for the preparation of a dental impression.

### Background of the Invention

Dental impression materials are known. Dental impression materials are commonly available as reactive multi-component materials provided in packages including two compartments or two separate containers that keep the components isolated from each other during storage. Once the components are mixed, a chemical reaction is initiated that turns the mixed composition into a hardened mass during the setting time. Moreover, the working time and the setting time of conventional dental impression materials are limited and depend on the rate of the curing reaction. Therefore, storage stability of a dental impression material depends on the separation of reactive components and necessitates mixing prior to use which needs to be done chairside immediately prior to use so that the dental impression may be completed during the working time of usually only a few minutes.

Devices have been developed for the automatic mixing and dispensing of multi-component dental impression materials in order to provide high precision with regard to the homogeneity of the mixture, and the ratio of the two components to be mixed. Accordingly, the components of the dental impression material are simultaneously supplied from separate material chambers to a mixer during application of the dental impression material, which mixes and then dispenses a mixed paste. The paste may be supplied from the mixer directly onto a dental impression tray for immediate placement in a patient's mouth.

Once the material components have come into contact with each other in the mixing chamber, the mixture of the material in the mixing chamber can only be stored for a short time because the mixed material will soon set inside the mixing chamber unless dispensed and used. Therefore, the dental practitioner may have to remove and replace mixers several times each day.

Dental impression materials may be silicone impression material curable in an addition or condensation reaction whereby addition silicones are most popular. Although conventional addition silicone impression materials provide good detail reproduction, excellent dimensional stability, little shrinkage on set, addition silicones are inherently hydrophobic and as such require moisture control for optimal use. Finally, addition silicones have only a poor tear resistance.

Dental impression material based on cross-linking polysiloxanes are known. For example, US-A-5849812 describes an addition-curing polyether dental impression material comprising (a) at least one polyether which has at least two optionally substituted vinyl and/or allyl end-groups, (b) an SiH component, (c) at least one platinum catalyst, (d) optional additives, and (e) organopolysiloxane with at least two alkenyl groups.

US-A 5137448 discloses a dental impression composition that is polymerizable by having an initiator activated by actinic light within the visible light range of 360 to 600 nanometers, which contains a compound having at least two terminal acrylate unsaturations and an organosilicone containing backbone.

### Summary of the Invention

It is the problem of the present invention to provide a dental impression material which is highly tolerant to moisture, which has adjustable working and setting times, and which has excellent tear resistance while providing at the same time good detail reproduction without adhesion to core build-up materials or composite restorations, excellent dimensional stability, and reduced shrinkage on set, and which may be provided as a single composition which does not need mixing prior to use.

Moreover, it is the problem of the present invention to provide a use of the dental impression material of the present invention.

The present invention provides a light-curable dental impression material comprising:

(a) a polymerizable polysiloxane resin composition comprising compounds of the following formula (I):

$$E\text{-}(L^1\text{-}Z)_n\text{-}L^2\text{-}E \qquad (I)$$

wherein

the E which may be the same or different, independently represent a monovalent group selected from a group

containing a polymerizable carbon-carbon double bond, a group containing a polysiloxane moiety, a $C_{2-20}$ alkoxy group, a $C_{2-20}$ thioalkyl group, and a RNH group, wherein R is a $C_{2-20}$ alkyl group;

$L^1$ which may be the same or different when more than one $L^1$ is present, represents a divalent group of the following formula (II):

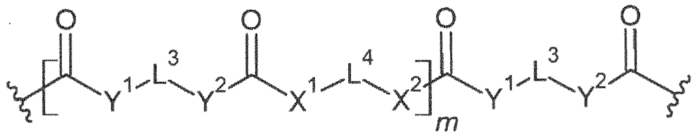

(II)

wherein

$L^3$ which may be the same or different when more than one $L^3$ is present, independently represents a divalent linker group which may be substituted with up to four substituents of the formula (III):

$$-L^1-E \qquad \text{(III)}$$

wherein $L^1$ and E are as defined above;
$L^4$ which may be the same or different when more than one $L^4$ is present, independently represents a divalent linker group which may be substituted with up to four substituents of the formula (III), wherein $L^1$ and E are as defined above;
$X^1$, $X^2$, $Y^1$, and $Y^2$,
which may be the same or different, and when more than one $X^1$, $X^2$, $Y^1$, or $Y^2$, is present, the $X^1$, $X^2$, $Y^1$, and $Y^2$ may be the same or different, independently represent an oxygen atom, a sulfur atom and a group NR', wherein R' is a hydrogen atom or a $C_{1-4}$ alkyl group;
m represents 0 or an integer of from 1 to 40;

Z represents a divalent linker group which may additionally be substituted with up to four substituents selected from polysiloxane groups and groups of the formula (III), wherein $L^1$ and E are as defined above;

$L^2$ represents a single bond or a divalent group of the formula (II), wherein $L^3$, $L^4$, $X^1$, $X^2$, $Y^1$, $Y^2$ and m are independently as defined for $L^1$;

n represents 0 or an integer of from 1 to 4;

provided that

- a compound of formula (I) contains at least one monovalent group E having a polymerizable carbon-carbon double bond,

- a compound of formula (I) contains at least one polysiloxane group, and

provided that
when n is 0, then $L^2$ is a divalent group of the formula (II);
(b) a particulate filler; and
(c) a photoinitiator.

Where m is greater than 1, then $L^3$, $L^4$, $X^1$, $X^2$, $Y^1$ and $Y^2$ may each be independently the same or different as defined above such that the repeating unit of formula (II) forms a block copolymer where the blocks may be the same, or random block copolymers, or alternate block copolymers, or sequential block copolymers. Further, the present invention provides a use of the light-curable dental impression material of the present invention for the preparation of a dental impression.

The present invention is based on the recognition that a specific composition of radically polymerizable polysiloxane compounds according to formula (I) may be used in a filled light-curable dental impression material given that such compounds contain at least one monovalent group E having a polymerizable carbon-carbon double bond, and at least one polysiloxane group so that the compounds have a low dynamic viscosity and may be cured in a radical polymerization reaction resulting in a cured material having limited adhesion to hard and soft tooth structure, core build-up materials or composite restorations.

The curing by radical polymerization allows to provide a convenient one component light-curable dental impression material which has high storage stability and which does not require mixing prior to use. Accordingly, the light-curable dental impression material may be provided as a ready-to-use dental tray. At the same time, the light-curable dental impression material provides, when cured, excellent tear resistance, good detail reproduction, excellent dimensional stability, and no shrinkage on set.

**Brief description of the Figures**

Fig. 1 shows a scheme of the synthesis procedure of compounds according to formula (I).

Fig. 2 shows a scheme of the synthesis procedure of macromonomers which may be used in combination with the compounds according to formula (I).

Fig. 3 shows a scheme of a reaction pathway towards high molecular weight crosslinkers having three or more polymerizable groups, which may be used in combination with the compounds according to formula (I).

Fig. 4 shows a scheme of a reaction pathway towards further macromonomers which may be used in combination with the compounds according to formula (I).

**Detailed Description of the Preferred Embodiments**

The terms "polymerization" and "polymerizable" relates to the combining or the capability to combine by covalent bonding of a large number of smaller molecules, such as monomers, to form larger molecules, that is, macromolecules or polymers. The monomers may be combined to form only linear macromolecules or they may be combined to form three-dimensional macromolecules, commonly referred to as crosslinked polymers. For example, monofunctional monomers form linear polymers, whereas monomers having at least two functional groups form crosslinked polymers also known as networks.

The term "$C_{2-20}$ alkylene group" according to the present invention represents a divalent $C_{2-20}$ hydrocarbon linker group. In particular, the linker group may be a hydrocarbon group which may be aliphatic and/or aromatic. Moreover, the linker group may be a straight-chain, branched and/or cyclic a hydrocarbon group. A $C_{2-20}$ hydrocarbon linker group may contain 1 to 8 heteroatoms selected from oxygen, nitrogen and sulphur, which divalent organic residue may be substituted with one or more substituents selected from the group consisting of a $C_{1-4}$ alkoxy groups, a hydroxyl group, a thiol group, and a $C_{6-14}$ aryl group. The $C_{2-20}$ alkylene group may be a linear or branched group. The hydrocarbon group may be substituted by 1 to 6 $C_{1-4}$ alkyl groups. Specific examples of the alkyl groups are methylene, ethylene, n-propylene, i-propylene, n-butylene, i-butylene or tert.-butylene. In a preferred embodiment, the hydrocarbon group of may contain 1 to 5 oxygen atoms in the hydrocarbon group in the form of aliphatic or aromatic ether bonds, keto groups, carboxylic acid groups, or hydroxyl groups. In case of an aliphatic group, the $C_{2-20}$ alkylene group may be a straight chain or branched alkylene group or a cycloalkylene group. In case of an aromatic group, the $C_{2-20}$ alkylene group may be an arylene group or a $C_{3-14}$ heteroarylene group. Specifically, it may be a divalent substituted or unsubstituted $C_{2-20}$ alkylene group, substituted or unsubstituted $C_{6-14}$ arylene group, substituted or unsubstituted $C_{3-20}$ cycloalkylene group, substituted or unsubstituted $C_{7-20}$ arylenealkylenearylene group. Furthermore, the $C_{2-20}$ alkylene group represents a saturated aliphatic $C_{2-20}$ hydrocarbon chain which may contain 2 to 4 oxygen atoms or nitrogen atoms, and which may be substituted by 1 to 6 $C_{1-4}$ alkyl groups, or the $C_{2-20}$ alkylene group may be a substituted or unsubstituted $C_{7-20}$ arylenealkylenearylene group which may be substituted by 1 to 6 $C_{1-4}$ alkyl groups.

"Actinic radiation" is any electromagnetic radiation that is capable of producing photochemical action and can have a wavelength of at least 150 nm and up to and including 1250 nm, and typically at least 300 nm and up to and including 750 nm.

The term "electron donor" as used herein means a compound capable of contributing electrons in a photochemical process. Suitable examples include organic compounds having heteroatoms with electron lone pairs, for example amine compounds.

The present invention provides a light-curable dental impression material. The light-curable dental impression material is preferably a one-pack composition packaged in a syringe or provided on a ready-to-use dental impression tray. Alternatively, the light-curable dental impression material of the present invention may also be a two-pack composition, in particular when formulated as a dual cure composition containing an additional redox initiator system.

When packaged in a syringe or provided on a ready-to-use dental impression tray, the composition must be

shielded from actinic light during storage.

The light-curable dental impression material of the present invention comprises a polymerizable polysiloxane resin composition comprising compounds of the formula (I) having a polymerizable moiety in combination with a polysiloxane moiety. The polymerizable polysiloxane resin composition comprises compounds of the formula (I) which are macromonomers. The macromonomers may be obtained by reacting a first reactant and a second reactant in a specific stoichiometric ratio so that an excess with regard to the molar amount of reactive groups of the second reactant is present, and using a third reactant in a specific ratio to end-cap any excess reactive end-groups.

The reaction of the first reactant and the second reactant is preferably an addition polymerization. However, a polycondensation is also possible.

The end-capping reaction of the third reactant with excess functional groups of the second reactant is preferably also an addition polymerization. However, a polycondensation is again possible.

In case of a polyaddition reaction, the formation of a urethane bond is preferred. Alternatively, the formation of urea or S-thiocarbamate linkages by the reaction of the first and second reactants is also possible. Accordingly, the first reactant may be a diol, or alternatively a diamine or a dithiol, and the second reactant may be a diisocyanate.

In case of polyfunctional reactants such as trifunctional reactants, branching may be introduced into the macromonomer. According to the present invention, it is possible to use a di-or polyalcohol as the first reactant and a di- or polyisocyanate as the second reactant whereby an isocyanate-terminated polyurethane prepolymer is formed which is endcapped with a monofunctional third reactant such as an alcohol as shown in Figs. 1. Alternatively, the use a di-or polyamine or di-or polythiol as the first reactant and a di- or polyisocyanate as the second reactant results in an isocyanate-terminated urea or S-carbamate prepolymer which is endcapped with a monofunctional third reactant such as an alcohol, amine or thiol compound.

However, according to the present invention, it is also possible to use a di-or polyisocyanate as the first reactant and a di- or polyalcohol as the second reactant whereby a hydroxyl-terminated prepolymer is formed which is end-capped with a monofunctional third reactant such as an isocyanate. Alternatively, it is possible to use a di-or polyisocyanate as the first reactant and a di- or polyamine or di- or polythiol as the second reactant whereby an amine or thiol-terminated prepolymer is formed which is end-capped with a monofunctional third reactant such as an isocyanate.

The first, second and third reactants may be mixtures of two or more different compounds. Any of the first, second and third reactants may contain one or more polysiloxane moieties. Preferably, the third component introduces a radical polymerizable moiety into the macromonomer.

According to a preferred embodiment, the light-curable dental impression material of the present invention further comprises additional polymerizable compounds, notably compounds which are compounds of formula (I), but lack any polysiloxane moiety. Specifically, the light-curable dental impression material of the present invention preferably comprises additional polymerizable macromonomers. The polymerizable macromonomers may be obtained by reacting a fourth reactant and a fifth reactant in a specific stoichiometric ratio so that an excess with regard to the molar amount of reactive groups of the fifth reactant is present, and using a sixth reactant in a specific ratio to end-cap any excess reactive end-groups.

The reaction of the fourth reactant and the fifth reactant is preferably an addition polymerization. However, a polycondensation is also possible. The end-capping reaction of the sixth reactant with excess functional groups of the fifth reactant is preferably also an addition polymerization. However, a polycondensation is again possible.

In case of a polyaddition reaction, the formation of a urethane bond is preferred. Alternatively, the formation of urea or S-thiocarbamate linkages by the reaction of the fourth and fifth reactants is also possible. Accordingly, the fourth reactant may be a diol, or alternatively a diamine or a dithiol, and the second reactant may be a diisocyanate.

In case of polyfunctional reactants, branching may be introduced into the macromonomer. According to the present invention, it is possible to use a di-or polyalcohol as the fourth reactant and a di- or polyisocyanate as the fifth reactant whereby an isocyanate-terminated prepolymer is formed which is endcapped with a monofunctional sixth reactant such as an alcohol as shown in Figs. 2. Alternatively, the use a di-or polyamine or di-or polythiol as the fourth reactant and a di- or polyisocyanate as the fifth reactant results in an isocyanate-terminated urea or S-carbamate prepolymer which is endcapped with a monofunctional third reactant such as an alcohol, amine or thiol compound.

However, according to the present invention, it is also possible to use a di-or polyisocyanate as the fourth reactant and a di- or polyalcohol as the fifth reactant whereby a hydroxyl-terminated polyurethane prepolymer is formed which is end-capped with a monofunctional sixth reactant such as an isocyanate. Alternatively, it is possible to use a di- or polyisocyanate as the fourth reactant and a di- or polyamine or di- or polythiol as the second reactant whereby an amine or thiol-terminated polyurea or poly-S-thiocarbamate prepolymer is formed which is end-capped with a monofunctional third reactant such as an isocyanate.

The fourth, fifth and sixth reactants may be mixtures of two or more different compounds. None of the first, second and third reactants may contain any polysiloxane moiety in case of the additional polymerizable macromonomers. Reactants which do not contain any polysiloxane moiety used for preparing a polymerizable polysiloxane resin composition comprising compounds of the formula (I) may be used for preparing the additional polymerizable macromonomers.

Preferably, the sixth component introduces a radical polymerizable moiety into the additional polymerizable macromonomer.

The polymerizable polysiloxane resin composition

The light-curable dental impression material of the present invention comprises a polymerizable polysiloxane resin composition comprising compounds of the following formula (I):

$$E-(L^1-Z)_n-L^2-E \qquad (I)$$

According to a preferred embodiment, the polymerizable polysiloxane resin composition has a total siloxane content in a range of from 10 to 40% wt/wt, more preferably in a range of from 15 to 35% wt/wt. The total siloxane content of the polymerizable polysiloxane resin composition may be determined by $^{29}$Si-NMR.

In a compound of formula (I), E which may be the same or different, independently represent a monovalent group selected from a group containing a polymerizable carbon-carbon double bond, a group containing a polysiloxane moiety, a $C_{2-20}$ alkoxy group, a $C_{2-20}$ thioalkyl group, and a RNH group, wherein R is a $C_{2-20}$ alkyl group.

According to a preferred embodiment, E is a group containing a polymerizable carbon-carbon double bond, preferably a (meth)acryloyl group, a (meth)acrylamide group or an allyl (meth)acrylamide group, more preferably a (meth)acryloyl group.

According to preferred embodiment, the monovalent groups E contain (meth)acrylate groups so that the total (meth)acrylate content of the polymerizable polysiloxane resin composition is in a range of from 0.20 to 0.50 mmol/g, more preferably from 0.25 to 0.45 mmol/g. The total (meth)acrylate content of the polymerizable polysiloxane resin composition is determined by using $^1$H-NMR.

According to a preferred embodiment, E is a group according to the following formula (IV):

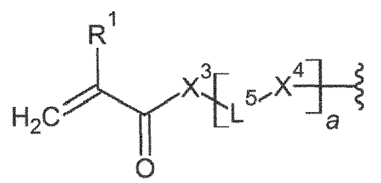

(IV)

wherein

- $R^1$ represents a hydrogen atom or a $C_{1-12}$ alkyl group;
- $X^3$ represents an oxygen atom, a sulfur atom or a group $NR^2$, wherein $R^2$ is a hydrogen atom, $C_{1-12}$ alkyl group, or an allyl group;
- $L^5$ is a divalent hydrocarbon linker group or polysiloxane containing linker group, preferably a $C_{2-20}$ alkylene group;
- $X^4$ represents an oxygen atom, a sulfur atom or a group $NR^3$, wherein $R^3$ is a hydrogen atom, or a $C_{1-12}$ alkyl group; and
- a represents an integer of from 1 to 20.

Preferably, $R^1$ represents a $C_{1-4}$ alkyl group, more preferably a methyl group.

According to a first embodiment, $X^3$ preferably represents an oxygen atom or a sulfur atom, more preferably an oxygen atom. According to a second embodiment, $X^3$ preferably represents a group $NR^2$, wherein $R^2$ is a hydrogen atom, $C_{1-12}$ alkyl group, or an allyl group.

Preferably, $X^4$ represents an oxygen atom or a sulfur atom, more preferably an oxygen atom.

$L^5$ is a divalent hydrocarbon linker group or polysiloxane containing linker group. A polysiloxane containing linker group may be a polysiloxane group or contain one or more hydrocarbon fragments in the main chain. Preferably, $L^5$ is a linear or branched $C_{2-3}$ alkylene group.

According to a preferred embodiment, the group $-[L^5-X^4]-$ is selected from the following groups:

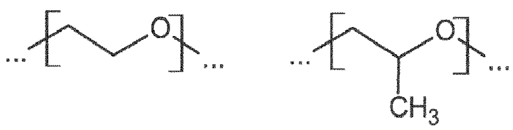

Preferably, a is an integer of from 1 to 10.

According to a preferred embodiment, E is a polysiloxane group of the following formula (V):

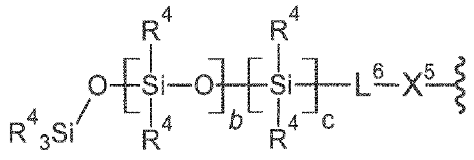

(V)

wherein

- $R^4$ which may be the same or different, independently represent a straight-chain, branched or cyclic alkyl group;
- $L^6$ is a divalent hydrocarbon linker group, preferably a $C_{2-20}$ alkylene group;
- $X^5$ represents an oxygen atom, a sulfur atom or a group $NR^5$, wherein $R^5$ is a hydrogen atom, or a $C_{1-12}$ alkyl group;
- b is 0 or an integer of from 1 to 1000; and
- c is 0 or 1.

According to a preferred embodiment, $R^4$ is a straight chain, optionally substituted $C_{1-12}$ alkyl group, preferably it is a straight chain optionally substituted $C_{1-6}$ alkyl group.

According to a preferred embodiment, $R^4$ is a branched, optionally substituted $C_{1-12}$ alkyl group, preferably it is a branched, optionally substituted $C_{1-6}$ alkyl group.

According to a preferred embodiment, $R^4$ is a cyclic, optionally substituted $C_{1-12}$ alkyl group, preferably it is a cyclic, optionally substituted $C_{1-6}$ alkyl group.

More preferably, $R^4$ is a methyl group.

Preferably, $X^5$ represents an oxygen atom or a sulfur atom, more preferably an oxygen atom.

Preferably, b is an integer of from 1 to 1000, more preferable it is an integer of from 1 to 100, and even more preferably it is an integer of from 1 to 10.

Preferably, c is 1.

According to a preferred embodiment, E is a linear optionally substituted $C_{2-20}$ alkoxy group.

According to a preferred embodiment, E is a branched optionally substituted $C_{2-20}$ alkoxy group.

Preferably, E is a linear optionally substituted $C_{2-15}$ alkoxy group, more preferably it is a linear unsubstituted $C_{2-15}$ alkoxy group, and even more preferably it is a linear unsubstituted $C_{12}$ alkoxy group.

According to a preferred embodiment, E is a linear optionally substituted $C_{2-20}$ thioalkyl group.

According to a preferred embodiment, E is a branched optionally substituted $C_{2-20}$ thioalkyl group.

Preferably, E is a linear optionally substituted $C_{2-15}$ thioalkyl group, more preferably it is a linear unsubstituted $C_{2-15}$ thioalkyl group.

According to a preferred embodiment, E is a RNH group, wherein R is a $C_{2-20}$ alkyl group.

Preferably, R is a linear optionally substituted $C_{2-20}$ alkyl group.

Preferably, R is a branched optionally substituted $C_{2-20}$ alkyl group.

Preferably, R is a linear optionally substituted $C_{2-15}$ alkyl group, more preferably it is a linear unsubstituted $C_{2-15}$ alkyl group, and even more preferably it is a linear unsubstituted $C_{2-6}$ alkyl group.

In a compound of formula (I), $L^1$ represents a divalent group of the following formula (II):

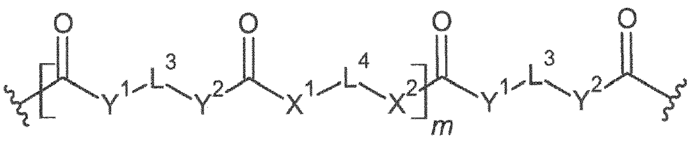

(II)

wherein

$L^3$ which may be the same or different when more than one $L^3$ is present, independently represents a divalent linker group which may be substituted with up to four substituents of the formula (III):

$-L^1-E$ (III)

wherein $L^1$ and E are as defined above;
$L^4$ which may be the same or different when more than one $L^4$ is present, independently represents a divalent linker group which may be substituted with up to four substituents of the formula (III), wherein $L^1$ and E are as defined above;
$X^1$, $X^2$, $Y^1$, and $Y^2$, which may be the same or different, and when more than one $X^1$, $X^2$, $Y^1$, or $Y^2$, is present, the $X^1$, $X^2$, $Y^1$, and $Y^2$ may be the same or different, independently represent an oxygen atom, a sulfur atom and a group NR', wherein R' is a hydrogen atom or a $C_{1-4}$ alkyl group;
m represents 0 or an integer of from 1 to 40.

Where m is greater than 1, then $L^3$, $L^4$, $X^1$, $X^2$, $Y^1$ and $Y^2$ may each be independently the same or different as defined above such that the repeating unit of formula (II) forms a block copolymer where the blocks may be the same, or random block copolymers, or alternate block copolymers, or sequential block copolymers.

According to a preferred embodiment, $L^3$ represents a $C_{2-20}$ alkylene group as defined above which may contain up to 10 heteroatoms in the main chain, which are selected from oxygen, sulfur or $NR^{\#}$, wherein $R^{\#}$ represents a hydrogen atom or a straight chain, branched or cyclic $C_{1-6}$ alkyl group.

According to a preferred embodiment, $L^3$ represents a group according to the following formula (VI):

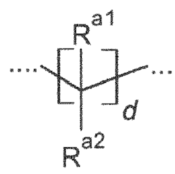

(VI)

wherein

| | |
|---|---|
| $R^{a1}$ and $R^{a2}$ | which may be the same or different, independently represent a hydrogen atom, a $C_{1-6}$ linear or branched alkyl group, a $C_{4-10}$ aryl group, a polymerizable double bond containing organic residue, preferably a (meth)acrylate group containing organic residue, a group of the following formula $[-X"L"]_mR^{a3}$, wherein X" represents O, S, or $NR^{a4}$ wherein $R^{a4}$ represents a hydrogen atom, an organic residue containing a polymerizable double bond, preferably an organic residue containing a (meth)acrylate group, a linear or branched $C_{1-6}$ alkyl group, or a $C_{4-10}$ aryl group, L" represents a $C_{1-6}$ linear or branched alkyl group, a $C_{4-10}$ aryl group, or a $SiR^{a5}_2$ group wherein $R^{a5}$ which may be the same or different, independently represent an organic residue containing a polymerizable double bond, preferably an organic residue containing a (meth)acrylate group, or a $C_{1-4}$ alkyl group, preferably a methyl group, m is an integer from 1 to 20, and $R^{a3}$ is an organic residue containing a polymerizable double bond, preferably an organic residue containing a (meth)acrylate group, a $C_{1-4}$ alkyl group, or a $C_{4-10}$ aryl group, and $R^{a1}$ and $R^{a2}$ may comprise a group of the formula (III); |
| d | is an integer of from 1 to 20, preferably an integer of from 1 to 10, and more preferably d is 6. |

Preferably, $L^3$ is selected from the following groups:

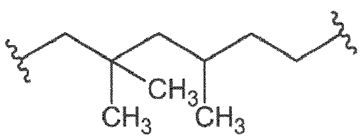

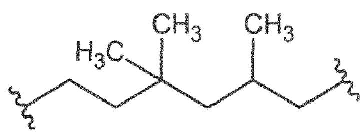

According to a preferred embodiment, $L^3$ represents a group according to the following formula (VII):

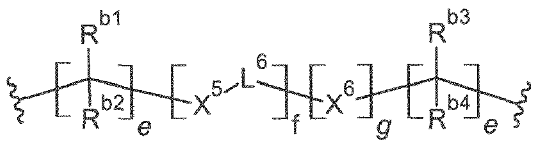

(VII)

wherein

$R^{b1}$, $R^{b2}$, $R^{b3}$ and $R^{b4}$ which may be the same or different, independently represent a hydrogen atom, a $C_{1-6}$ linear or branched alkyl group, a $C_{4-10}$ aryl group, a polymerizable double bond containing organic residue, preferably a (meth)acrylate group containing organic residue, a group of the following formula $[-X'''L''']_mR^{b5}$, wherein X''' represents O, S, or $NR^{b6}$ wherein $R^{b6}$ represents a hydrogen atom, an organic residue containing a polymerizable double bond, preferably an organic residue containing a (meth)acrylate group, a linear or branched $C_{1-6}$ alkyl group, or a $C_{4-10}$ aryl group, L''' represents a $C_{1-6}$ linear or branched alkyl group, a $C_{4-10}$ aryl group, or a $SiR^{b7}_2$ group wherein $R^{b7}$ which may be the same or different, independently represent an organic residue containing a polymerizable double bond, preferably an organic residue containing a (meth)acrylate group, or a $C_{1-4}$ alkyl group, preferably a methyl group, m is an integer from 1 to 20, and $R^{b5}$ is an organic residue containing a polymerizable double bond, preferably an organic residue containing a (meth)acrylate group, a $C_{1-4}$ alkyl group, or a $C_{4-10}$ aryl group, and $R^{b1}$, $R^{b2}$, $R^{b3}$,and $R^{b4}$ may comprise a group of the formula (III);

$X^5$ and $X^6$ which may be the same or different, and when more than one $X^5$ or $X^6$, are present, the $X^5$ and $X^6$ may be the same or different, independently represent an oxygen atom, a sulfur atom and a group $NR^N$, wherein $R^N$ is a hydrogen atom or a $C_{1-4}$ alkyl group;

$L^6$ is a divalent hydrocarbon linker group, preferably a $C_{2-20}$ alkylene group;

e is an integer of from 1 to 10;

f is an integer of from 1 to 100; specifically 1 to 40 or 40 to 100 and

9 is 0 or 1.

Preferably, $L^6$ is a linear or branched $C_{2-3}$ alkylene group.

According to a preferred embodiment, the group $-[L^6-X^5]-$ is selected from the following groups:

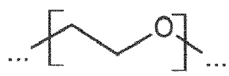 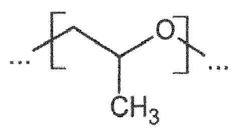

According to a preferred embodiment, $L^3$ represents a group according to the following formula (VIII):

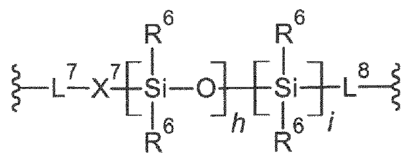

(VIII)

wherein

$R^6$ which may be the same or different, independently represent a straight-chain, branched or cyclic alkyl group;

$X^7$ represent an oxygen atom, a sulfur atom and a group $NR^{N1}$, wherein $R^{N1}$ is a hydrogen atom or a $C_{1-4}$ alkyl group;

$L^7$ and $L^8$ is a divalent hydrocarbon linker group, preferably a $C_{2-20}$ alkylene group;

h is an integer of from 1 to 40;

i is 0 or 1.

$L^3$ may also be a polysiloxane group of the following formula (VIIIa), (VIIIb), or (VIIIc)

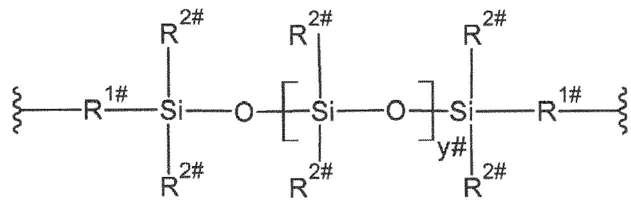

(VIIIa),

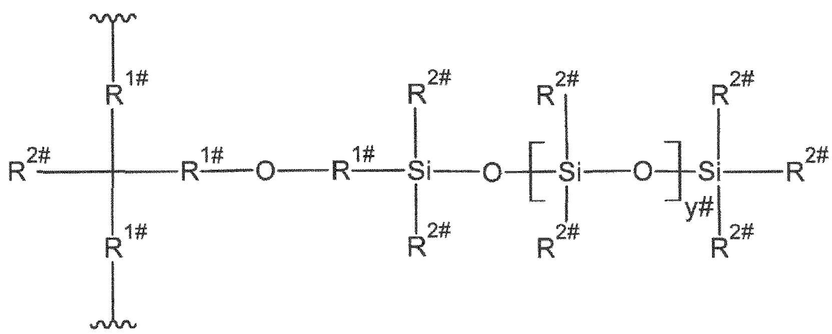

(VIIIb),

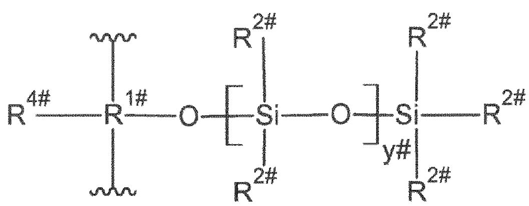

(VIIIc),

wherein

$R^{1\#}$ is an alkylene having 1 to 8 carbon atoms;
$R^{2\#}$ is an alkyl having from 1 to 4 carbon atoms;
$R^{4\#}$ is an alkyl, alkoxy or a cycloalkyl group;
$y^{\#}$ is an integer from 5 to 20.

According to a preferred embodiment, $R^6$ is a straight chain, optionally substituted $C_{1-12}$ alkyl group, preferably it is a straight chain optionally substituted $C_{1-6}$ alkyl group.
According to a preferred embodiment, $R^6$ is a branched, optionally substituted $C_{1-12}$ alkyl group, preferably it is a branched, optionally substituted $C_{1-6}$ alkyl group.
According to a preferred embodiment, $R^6$ is a cyclic, optionally substituted $C_{1-12}$ alkyl group, preferably it is a cyclic, optionally substituted $C_{1-6}$ alkyl group.
More preferably, $R^6$ is a methyl group.
Preferably, $X^7$ represents an oxygen atom.
Preferably, $L^7$ and $L^8$ which may be the same or different, independently represent a linear or branched $C_{2-3}$ alkylene group.
More preferably, $L^7$ and $L^8$ which may be the same or different, independently are selected from the following groups:

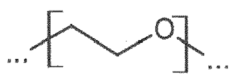 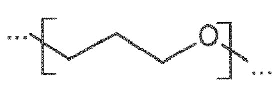 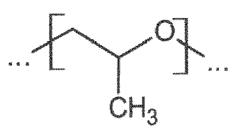

Preferably, h is an integer of from 1 to 20, more preferably it is an integer of from 1 to 10.
Preferably, i is 1.
According to a preferred embodiment, $L^4$ may represent any group as defined according to $L^3$.
According to a preferred embodiment, $L^4$ represents a group according to the following formula (IX):

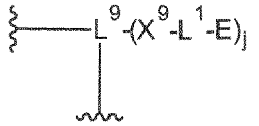

(IX)

wherein

| | |
|---|---|
| E and $L^1$ | are defined as above; |
| $L^9$ | is a divalent hydrocarbon linker group, preferably a $C_{2-20}$ alkylene group; |
| $X^9$ | when more than one $X^9$ is present the $X^9$ groups may be the same or different, independently represent an oxygen atom, a sulfur atom and a group $NR^{N2}$, wherein $R^{N2}$ is a hydrogen atom or a $C_{1-4}$ alkyl group; |
| j | is an integer of from 1 to 4. |

Preferably, $X^9$ is an oxygen atom or a sulfur atom, more preferably it is an oxygen atom.
Preferably, j is 1 or 2, more preferably it is 2.
According to a preferred embodiment, the group according to formula (IX) is selected from the following groups:

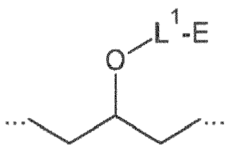 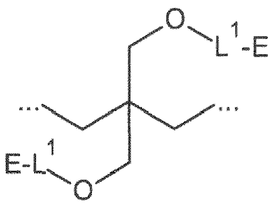

In a group of formula (II), $X^1$ and $X^2$ which may be the same or different, and when more than one $X^1$, and $X^2$ is present, the $X^1$, and $X^2$ may be the same or different, independently represent an oxygen atom, a sulfur atom and a group NR', wherein R' is a hydrogen atom or a $C_{1-4}$ alkyl group. Preferably, $X^1$ and $X^2$ represent an oxygen atom.
In a group of formula (II), $Y^1$ and $Y^2$ which may be the same or different, and when more than one $Y^1$, and $Y^2$ is present, the $Y^1$, and $Y^2$ may be the same or different, independently represent an oxygen atom, a sulfur atom and a group NR', wherein R' is a hydrogen atom or a $C_{1-4}$ alkyl group. Preferably, $Y^1$ and $Y^2$ represent a group NR', wherein R' is a hydrogen atom or a $C_{1-4}$ alkyl group. More preferably, $Y^1$ and $Y^2$ represent a group NR', wherein R' is a hydrogen atom.
In a group of formula (II), m represents 0 or an integer of from 1 to 40.
In a preferred embodiment m is 0.
In a preferred embodiment m is an integer of from 1 to 10.
Preferably, m is 1 or 2.
In a compound of formula (I), Z represents any group as defined according to $L^3$.
In a preferred embodiment, Z represents any group as defined according to $L^4$.
Preferably, Z represents a group according to formula (IX).
In a compound of formula (I), $L^2$ represents any group as defined according to $L^3$ or $L^4$. Preferably, $L^2$ represents any group as defined according to $L^3$.
Preferably, $L^2$ is selected from the following groups:

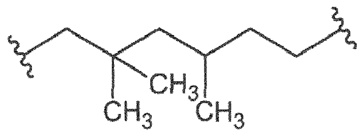

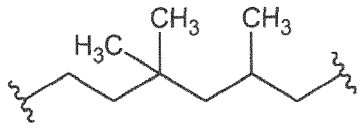

In a preferred embodiment, $L^2$ represents a single bond.

In a compound of formula (I), n represents 0 or an integer of from 1 to 4.

Ina preferred embodiment, n is >0.

In a preferred embodiment, n is 0 and $L^2$ is a divalent group of formula (II), wherein m is 0.

In a preferred embodiment, n= 0 and $L^2$ represents a divalent group of the formula (II), wherein

$L^3$ is a divalent $C_{1-12}$ hydrocarbon group or a polysiloxane group;
$L^4$ which may be the same or different when more than one $L^4$ is present, independently represents a divalent linker group which may be substituted with up to four substituents of the formula (III), wherein $L^1$ and E are as defined above;
$X^1$ and $X^2$ are oxygen atoms,
$Y^1$ and $Y^2$ are NH groups, and
m is an integer of from 1 to 40.

In a preferred embodiment, n is >0 and $L^2$ represents a divalent group of the formula (II), wherein

$L^3$ is a divalent $C_{1-12}$ hydrocarbon group or a polysiloxane group;
$L^4$ which may be the same or different when more than one $L^4$ is present, independently represents a divalent linker group which may be substituted with up to four substituents of the formula (III), wherein $L^1$ and E are as defined above;
$X^1$ and $X^2$ are oxygen atoms,
$Y^1$ and $Y^2$ are NH groups, and
m is an integer of from 1 to 40.

According to a preferred embodiment, $L^1$ and $L^2$ independently represent a divalent group of the formula (II), wherein
$L^3$ is a divalent $C_{1-12}$ hydrocarbon group or a polysiloxane group;

$L^4$ which may be the same or different when more than one $L^4$ is present, independently represents a divalent linker group which may be substituted with up to four substituents of the formula (III), wherein $L^1$ and E are as defined above;
$X^1$ and $X^2$ are oxygen atoms,
$Y^1$ and $Y^2$ are NH groups,
m is an integer of from 1 to 40; and
Z is a divalent $C_{1-12}$ hydrocarbon group.

According to a specific embodiment, the compound of formula (I) is a compound of any one of the following formulae:

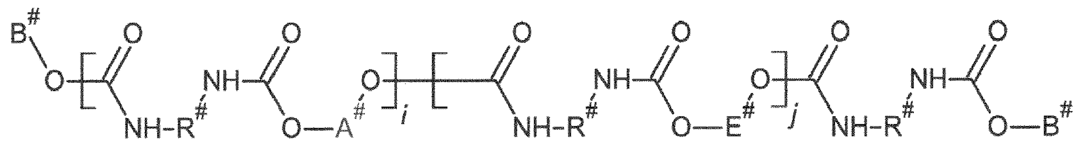

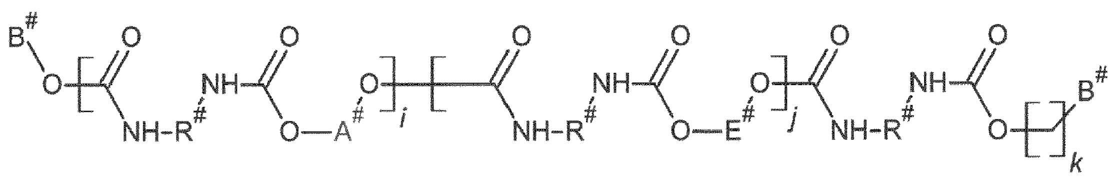

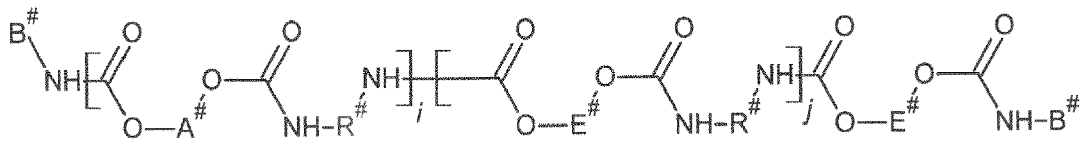

wherein
$B^{\#}$ is

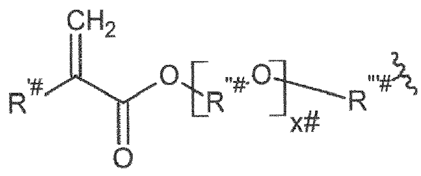

$A^{\#}$ is

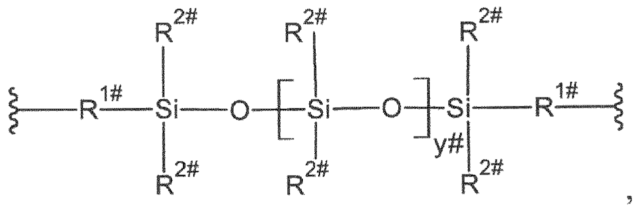

or

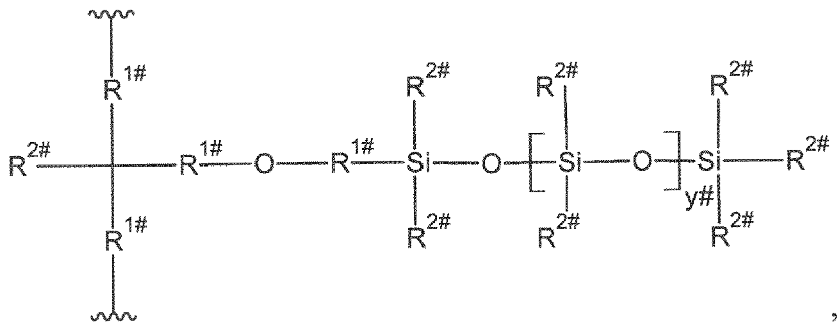

or

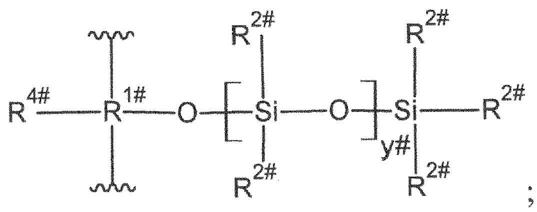

E is

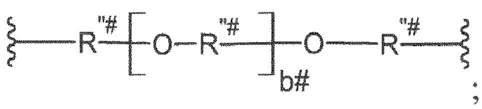

$R^{\#}$ is an alkylene having from 2 to 25 carbon atoms;

R'# is H or $CH_3$;
R"# is an alkylene having from 2 to 15 carbon atoms;
$R^{1\#}$ is an alkylene having 1 to 8 carbon atoms;
$R^{2\#}$ is an alkyl having from 1 to 4 carbon atoms;
$R^{4\#}$ is an alkyl, alkoxy or a cycloalkyl group;
i and j are independently an integer from 1 to 20;
$x^{\#}$ is an integer from 2 to 10;
$y^{\#}$ is an integer from 5 to 20;
$b^{\#}$ is an integer from 50 to 100; and
k is an integer from 5 to 15.

According to a preferred embodiment, the compound of formula (I) has a molecular weight of 100 to 10.000 Da, more preferably 300 to 2000 Da.

Preferably, a compound of formula (I) has a dynamic viscosity in the range of from 0.001 to 100 Pas, more preferably 0.1 to 10 Pas. The dynamic viscosity is a measure of the internal resistance of a fluid to flow. The dynamic viscosity can be measured with various types of viscometers and rheometers at a temperature of 25 °C.

According to a preferred embodiment, wherein $-L^1$-Z- represents a divalent group of the formula (II), which is obtainable by reacting a diisocyanate compound, a diol compound, and a polyol compound having at least three hydroxyl groups.

According to a preferred embodiment, $L^2$ represents a divalent group of the formula (II), which is obtainable by reacting a diisocyanate compound and a diol compound.

According to a preferred embodiment, the polymerizable polysiloxane resin mixture is obtainable by reacting a mixture comprising:

(a) x equivalents of one or more di- or polyol compounds of the following formula (X):

$$HO\text{-}L^4(OH)_l \qquad (X)$$

wherein

$L^4$ is an (l+1)-valent linker group; and
l is an integer of from 1 to 5;

(b) y equivalents of one or more compounds of the following formula (XI):

$$OCN\text{-}L^3NCO \qquad (XI)$$

wherein
$L^3$ is a divalent linker group; and
(c) z equivalents of one or more compounds of the following formula (XII):

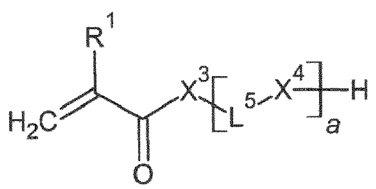

(XII)

wherein

- $R^1$ represents a hydrogen atom or a $C_{1-12}$ alkyl group;
- $X^3$ represents an oxygen atom, a sulfur atom or a group $NR^2$, wherein $R^2$ is a hydrogen atom, $C_{1-12}$ alkyl group, or an allyl group;
- $L^5$ is a divalent hydrocarbon linker group, preferably a $C_{2-20}$ alkylene group; and
- $X^4$ represents an oxygen atom, a sulfur atom or a group $NR^3$, wherein $R^3$ is a hydrogen atom, or a $C_{1-12}$ alkyl group,
- a represents an integer of from 1 to 20,

wherein $0.05 \leq x/y \leq 0.66$, and $2y - \overline{f}x \leq z \leq 1.5(2y - \overline{f}x)$,
wherein x, y, and z are the molar equivalents of components (a), (b) and (c) and $\overline{f}$ is the mean hydroxyl functionality of component (a):

$$\overline{f} = \sum_{l=1}^{3} \frac{x_l}{x}(l+1)$$

wherein l is as defined in formula (X) and $x_l/x$ is the molar fraction of the compounds having a hydroxyl functionality of l+1.

The mixture contains x molar equivalents of component (a), y molar equivalents of component (b), and z molar equivalents of component (c).

The molar equivalents are adjusted so that $0.05 \leq x/y \leq 0.66$, and $2y - f\,x \leq z \leq 1.5(2y - f\,x)$, wherein f is the mean hydroxyl functionality of component (a) defined by the following formula:

$$\overline{f} = \sum_{l=1}^{3} \frac{x_l}{x}(l+1)$$

wherein l is as defined in formula (X) and $x_l/x$ is the molar fraction of the compounds having an hydroxyl functionality of l+1.

Accordingly, the molar equivalents x of component (a) depend on the functionality of the one or more di- and/or polyhydroxyl compounds contained in component (a). According to a preferred embodiment, l is 1. When l is 1, then f is 2. According to a further preferred embodiment, the mixture contains one compound of formula (I), preferably wherein l is 1. As a result, compounds according to formula (I) are linear.

According to the present invention, the molar equivalent y is larger than the molar equivalent x in that $0.05 \leq x/y \leq 0.66$. Given that y>x and depending on the molar ratio of r'=x/y, the polymerization degree (Pa) increases according to Pa =(1+r')/(1-r'). In case x/y > 0.66, the viscosity of the polymerizable composition may become excessively large so that large amounts of a solvent or reactive diluent are required for providing a light-curable dental impression material of the present invention. In case x/y < 0.05, the polymerizable composition contains an excess of reaction products between component (b) and component (c) whereby the mechanical properties of the light-curable dental impression material of the present invention are deteriorated.

The mixture contains z molar equivalents of a one or more and chain terminating compounds (c). The amount of z is selected according to the present invention so that $2y - f\,x \leq z \leq 1.5(2y - fx)$, wherein f is the mean hydroxyl functionality of component (a) defined above. Preferably, z is 2y - f x. In case x < 2y - f x, then the content of end groups in the macromers contained in the polymerizable mixture of the present invention may be reduced which is not preferable in view of the mechanical properties of the dental composite of the present invention. In case z>1.5(2y - f x), the excess of chain terminating compounds may compete with the reaction of component (a) and component (b) and interfere with the macromer formation.

The average molecular weight $\overline{M}$ of the polymerizable mixture may be estimated according to the following formula:

$$\overline{M} = x\,\overline{M_{(a)}} + y\,\overline{M_{(b)}} + z\,\overline{M_{(c)}}$$

In the above formula $\overline{M_{(a)}}$ is the average molecular weight of component (a), $\overline{M_{(b)}}$ is the average molecular weight of component (b), and $\overline{M_{(c)}}$ is the average molecular weight of component (c).

According to an alternative embodiment, the polymerizable polysiloxane resin mixture is obtainable by reacting a mixture comprising:

(a') x equivalents of one or more di- or polyisocyanate compounds of the following formula (X'):

$$OCN\text{-}L^{4'}(NCO)_{l'} \qquad (X')$$

wherein

$L^{4'}$ is an (l'+1)-valent linker group; and
l' is an integer of from 1 to 5;

(b') y equivalents of one or more diol compounds of the following formula (XI'):

$$HO\text{-}L^{3'}OH \qquad (XI')$$

wherein
$L^{3'}$ is a divalent linker group; and
(c') z equivalents of one or more compounds of the following formula (XII'):

(XII') 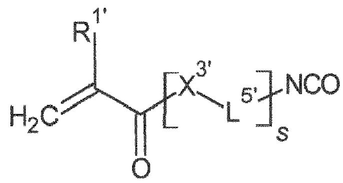

wherein

$R^{1'}$ represents a hydrogen atom or a $C_{1-12}$ alkyl group;
$X^{3'}$ represents an oxygen atom, a sulfur atom or a group $NR^2$, wherein $R^2$ is a hydrogen atom, $C_{1-12}$ alkyl group, or an allyl group;
$L^{5'}$ is a divalent hydrocarbon linker group, preferably a $C_{2-20}$ alkylene group;
s is 0 or an integer of from 1 to 6; and

wherein $0.05 \leq x/y \leq 0.66$, and $2y - \overline{f}x \leq z \leq 1.5(2y - \overline{f}x)$,
wherein x, y, and z are the molar equivalents of components (a'), (b') and (c') and $\overline{f}$ is the mean isocyanate functionality of component (a):

$$\overline{f} = \sum_{l=1}^{3} \frac{x_l}{x} (l + 1)$$

wherein l is l' as defined in formula (X') and $x_l/x$ is the molar fraction of the compounds having an isocyanate functionality of l'+1.

The mixture contains x molar equivalents of component (a'), y molar equivalents of component (b'), and z molar equivalents of component (c').

The molar equivalents are adjusted so that $0.05 \leq x/y \leq 0.66$, and $2y - f x \leq z \leq 1.5(2y - f x)$, wherein f is the mean isocyanate functionality of component (a) defined by the following formula:

$$\overline{f} = \sum_{l=1}^{3} \frac{x_l}{x} (l + 1)$$

wherein l is as defined in formula (X) and xi/x is the molar fraction of the compounds having an hydroxyl functionality of l+1.

Accordingly, the molar equivalents x of component (a) depend on the functionality of the one or more di- and/or polyhydroxyl compounds contained in component (a). According to a preferred embodiment, l is 1. When l is 1, then f is 2. According to a further preferred embodiment, the mixture contains one compound of formula (I), preferably wherein l is 1. As a result, compounds according to formula (I) are linear.

According to the present invention, the molar equivalent y is larger than the molar equivalent x in that $0.05 \leq x/y \leq 0.66$. Given that y>x and depending on the molar ratio of r'=x/y, the polymerization degree (Pa) increases according to Pa =(1+r')/(1-r'). In case x/y > 0.66, the viscosity of the polymerizable composition may become excessively large so that large amounts of a solvent or reactive diluent are required for providing a light-curable dental impression material of the present invention. In case x/y < 0.05, the polymerizable composition contains an excess of reaction products between component (b) and component (c) whereby the mechanical properties of the light-curable dental impression material of the present invention are deteriorated.

The mixture contains z molar equivalents of a one or more and chain terminating compounds (c). The amount

of z is selected according to the present invention so that $2y - f x \leq z \leq 1.5(2y - fx)$, wherein f is the mean isocyanate functionality of component (a) defined above. Preferably, z is $2y - f x$. In case $x < 2y - f x$, then the content of end groups in the macromers contained in the polymerizable mixture of the present invention may be reduced which is not preferable in view of the mechanical properties of the dental composite of the present invention. In case $z > 1.5(2y - f x)$, the excess of chain terminating compounds may compete with the reaction of component (a) and component (b) and interfere with the macromer formation.

The average molecular weight $\overline{M}$ of the polymerizable mixture may be estimated according to the following formula:

$$\overline{M} = x\,\overline{M_{(a)}} + y\,\overline{M_{(b)}} + z\,\overline{M_{(c)}}$$

In the above formula $\overline{M_{(a)}}$ is the average molecular weight of component (a'),

$\overline{M_{(b)}}$ is the average molecular weight of component (b'), and $\overline{M_{(c)}}$ is the average molecular weight of component (c').

Preferably, the light-curable dental impression material comprises 2 to 95 percent by weight, based on the total weight of the light-curable dental impression material, of a polymerizable polysiloxane resin composition comprising compounds of formula (I). More preferably, the light-curable dental impression material comprises 4 to 20 percent by weight, based on the total weight of the light-curable dental impression material, of compounds of formula (I).

Additionally, the light-curable dental impression material may preferably comprise up to 75 percent by weight, based on the total weight of the light-curable dental impression material, of polymerizable compounds of formula (I), but lack any polysiloxane moiety. Preferably, the light-curable dental impression material may comprises 10 to 50 percent by weight, based on the total weight of the light-curable dental impression material, of such polymerizable compounds of formula (I) lacking any polysiloxane moiety.

In the preparation of the polymerizable compounds of formula (I), coupling agents may be used. Examples of suitable coupling agents are bismuth compounds such as bismuth 2-ethyl hexanoate (K-KAT 348), tin compounds such as dibutyl tin dioctanoate, and tertiary amines such as 1,4-diazabicyclo[2.2.2]octane (DABCO).

<u>The filler</u>

The light-curable dental impression material of the present invention comprises a filler. The filler is a particulate filler which has preferably a mean particle size in the range of from 0.05 to 5 μm as measured, for example, by electron microscopy or by using a conventional laser diffraction particle sizing method as embodied by a MALVERN Mastersizer S or MALVERN Mastersizer 3000 apparatus. The particulate filler may be a multimodal particulate filler representing a mixture of two or more particulate fractions having different average particle sizes. The particulate reactive filler may also be a mixture of particles of different chemical composition.

Preferably, the photocurable dental impression material comprises 10 to 60 percent by weight, more preferably 20 to 50 percent by weight, based on the total weight of the dental impression material of a filler.

The specific type of filler is not particularly limited. In order to achive a usable depth of cure, preferred are fillers with a refrective index comparable to the one of the polymerisable resin mixture. Accordingly, any toxicologically acceptable inorganic, especially hydrophobic fillers may be employed such as silicas, aluminas, magnesias, titanias, inorganic salts, metallic oxides and glasses.

The filler may be a mixtures of different fillers such as silicone dioxides including crystalline forms, in particular particulate quartz, amorphous silicon dioxides, in particular diatomaceous earth, and silanated fumed silica.

The viscosity and thixotropicity of the uncured as well as the physical properties of the cured compositions may be controlled by varying the sizes and surface areas of the filler.

The filler may be surface treated with one or more silanating agents. Preferred silanating agents include those having at least one polymerizable double bond and at least one group that easily hydrolyses with water. Examples of such agents include 3-methacryloxypropyltrimethoxysilane, 3-methacryloxypropyldimethoxy-monochlorosilane, 3-methacryloxypropyldichloromonomethoxysilane, methacryloxypropyltri-chlorosilane, 3-methacryloxypropyldichloromonomethyl-silane, 3- methacryloxypropylmonochlorodimethylsilane, and mixtures thereof.

Preferred filler are fumed silica, quartz, cristobalite, calcium silicate, diatomaceous earth, zirconium silicate, montmorillonite such as bentonite, zeolite, including molecular sieves such as sodium aluminium silicate, metal oxide powder such as aluminium or zinc oxide or their mixed oxides, barium sulphate, calcium carbonate, plaster, and glass powder.

Preferably, the light-curable dental impression material comprises 5 to 50 percent by weight, based on the total

weight of the light-curable dental impression material, of a particulate filler. More preferably, the filler is contained in an amount of from 10 to 45 percent by weight based on the total weight of the light-curable dental impression material.

The photoinitiator.

The light-curable dental impression material of the present invention comprises a photoinitiator. The photoinitiator generates free radicals upon exposure to actinic light. Free radicals may be typically produced by either of two pathways:

(1) a photoinitiator compound undergoes excitation by energy absorption with subsequent decomposition of the compound into one or more radicals (Norrish type I), or
(2) a photoinitiator compound undergoes excitation and the excited photoinitiator compound interacts with a second compound by either energy transfer or a redox reaction to form free radicals from any of the compounds (Norrish type II).

According to the present invention any compound or system capable of initiating the polymerization of the mixture of polymerizable silicone compounds according to the present invention may be used.

For example, a suitable photoinitiator system may be in the form of a binary or tertiary system. A binary systems may include a photoinitiator and an electron donor compound, and a tertiary system may include an iodonium, sulfonium or phosphonium salt, a photoinitiator, and an electron donor compound, as for example described in US 5,545,676.

Suitable photoinitiators are monoketones and diketones that absorb some light within a range of about 400 nm to about 520 nm (preferably, about 450 nm to about 500 nm). Particularly suitable compounds include alpha diketones that have some light absorption within a range of about 400 nm to about 520 nm (even more preferably, about 450 to about 500 nm). Examples include camphor quinone, benzil, furil, 3,3,6,6-tetramethylcyclo-hexanedione, phenanthraquinone, 1-phenyl-1,2-propanedione and other 1-aryl-2-alkyl-1,2-ethanediones, and cyclic alpha diketones.

Moreover, suitable photoinitiators are compounds of the following formula (III) as disclosed in EP 3231413 A1 and EP 3153150 A1:

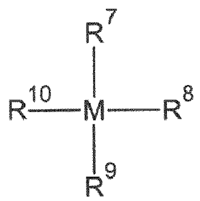

(XIII)

In a compound of formula (XIII), M is Ge or Si.

Moreover, in a compounds of formula (XIII), $R^7$, $R^8$ and $R^9$ may be the same or different, independently represent an organic group. Preferably, $R^7$ and $R^8$ independently from each other represent a substituted or unsubstituted hydrocarbyl or hydrocarbylcarbonyl group, and $R^9$ represents a substituted or unsubstituted hydrocarbyl group. The hydrocarbyl group may be an alkyl group, a cycloalkyl group, a cycloalkylalkyl group, an arylalkyl group or an aryl group. An alkyl group may be linear $C_{1-20}$ or branched $C_{3-20}$ alkyl group, typically a linear $C_{1-8}$ or branched $C_{3-8}$ alkyl group. Examples for $C_{1-16}$ alkyl groups can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. A cycloalkyl group may be a $C_{3-20}$ cycloalkyl group, typically a $C_{3-8}$ cycloalkyl group. Examples of the cycloalkyl group can include those having 3 to 6 carbon atoms, for example, cyclopropyl, cyclobutyl, cyclopentyl and cyclohexyl. A cycloalkylalkyl group may have 4 to 20 carbon atoms and may include a combination of a linear or branched alkyl group having 1 to 6 carbon atoms and a cycloalkyl group having 3 to 14 carbon atoms. Examples of the cycloalkylalkyl(-) group can for example, include methylcyclopropyl methylcyclobutyl, methylcyclopentyl, methylcyclohexyl, ethylcyclopropyl, ethylcyclobutyl, ethylcyclopentyl, ethylcyclohexyl, propylcyclopropyl, propylcyclobutyl, propylcyclopentyl, propylcyclohexyl. An arylalkyl group may be a $C_{7-20}$ arylalkyl group, typically a combination of a linear or branched alkyl group having 1 to 6 carbon atoms and an aryl group having 6 to 10 carbon atoms. Specific examples of an arylalkyl group are a benzyl group or a phenylethyl group. An aryl group can include aryl groups having 6 to 10 carbon atoms. Examples of the aryl group are phenyl and naphtyl.

The hydrocarbylcarbonyl groups of $R^7$ and $R^8$ represent acyl groups ($R_{org}$-(C=O)-) in which the organic residue $R_{org}$ is a hydrocarbyl residue as defined above.

Compound of formula (XIII) may contain one or two hydrocarbylcarbonyl groups, that is either one of $R^7$ and $R^8$ is a hydrocarbylcarbonyl group, or both $R^7$ and $R^8$ are hydrocarbylcarbonyl groups. Preferably, compound of formula (XIII) contains one hydrocarbylcarbonyl group. Preferably, the hydrocarbylcarbonyl group is an arylcarbonyl group, more preferably a benzoyl group. Preferably, $R^7$ and $R^8$ are independently selected from the group consisting of alinear $C_{1-6}$ or branched $C_{3-6}$ alkyl group, and a phenyl or benzoyl group which may optionally be substituted by one to three substitutents selected from halogen atoms, a nitro group, a $C_{1-4}$ alkoxy group and a $-NR^xR^y$ group wherein $R^x$ and $R^y$ independently from each other represent a $C_{1-4}$ alkyl group, and $R^9$ is a linear or branched $C_{3-6}$ alkyl group or a phenyl group. Most preferably, $R^7$ and $R^8$ are independently selected from the group of a linear $C_{1-4}$ or branched $C_3$ or $C_4$ alkyl group, and a phenyl or benzoyl group which may optionally be substituted with one substituent selected from the group consisting of selected from a halogen atom, a nitro group, a $C_{1-4}$ alkoxy group and a $-NR^xR^y$ group wherein $R^x$ and $R^y$ independently from each other represent a $C_{1-4}$ alkyl group, and $R^5$ is a linear $C_{1-4}$ or branched $C_3$ or $C_4$ alkyl group.

Moreover, in a compounds of formula (XIII), $R^{10}$ represents a hydrogen atom, an organic or organometallic group, provided that when $R^{10}$ is a hydrogen atom, the initiator system further comprises a sensitizer compound having a light absorption maximum in the range from 300 to 600 nm.

According to a first preferred embodiment, $R^{10}$ represents a group of the following formula (XIV):

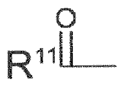

(XIV)

wherein $R^{11}$

(i) is a group of the following formula (XV):

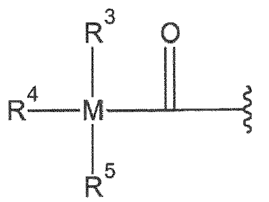

(XV)

wherein M, $R^7$, $R^8$ and $R^9$ have the same meaning as defined above for formula (XIII), whereby the compound of formula (XIII) may be symmetrical or unsymmetrical; or

(ii) is a group of the following formula (XVI):

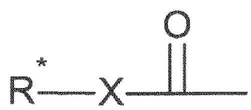

(XVI)

wherein

X represents a single bond, an oxygen atom or a group $NR^{X1}$, wherein $R^{X1}$ represents a substituted or unsubstituted hydrocarbyl group;

R* represents a substituted or unsubstituted hydrocarbyl group, a trihydrocarbylsilyl group, a mono(hydrocarbylcarbonyl)dihydrocarbylsilyl group or a di(hydrocarbylcarbonyl)monohydrocarbylsilyl group; or

(iii) when M is Si, R may be a substituted or unsubstituted hydrocarbyl group.

For R* of formula (XVII) being a trihydrocarbylsilylgroup, a mono(hydrocarbylcarbonyl)-dihydrocarbylsilyl group or a di(hydrocarbylcarbonyl)monohydrocarbylsilyl group, each of the hydrocarbyl and hydrocarbylcarbonyl groups has

the same meaning as defined for $R^7$, $R^8$ and $R^9$ and is independently selected therefrom.

In formula (XVI), R' has the same meaning as defined for $R^9$ and is independently selected therefrom.

According to a second preferred embodiment, $R^{10}$ represents a hydrogen atom. Accordingly, the initiator system further comprises a sensitizer compound. The sensitizer compound is preferably an alpha-diketone sensitizer compound having a light absorption maximum in the range from 300 to 500 nm. The alpha-diketone sensitizer is capable of absorbing visible light and forming a photoexcitation complex with a hydrogen donating compound of formula (XIII). The alpha-diketone photoinitiator compound may be selected from camphorquinone, 1,2-diphenylethane-1,2-dione (benzil), 1,2-cyclohexanedione, 2,3-pentanedione, 2,3-hexanedione, 3,4-hexanedione, 2,3-heptanedione, 3,4-heptanedione glyoxal, biacetyl, 3,3,6,6-tetramethylcyclohexanedione, 3,3,7,7-tetramethyl-1,2-cycloheptanedione, 3,3,8,8-tetramethyl-1,2-cyclooctanedione; 3,3,18,18-tetramethyl-1,2-cyclooctadecanedione; dipivaloyl; furil, hydroxybenzil, 2,3-butanedione, 2,3-octanedione, 4,5-octanedione, and 1-phenyl-1,2-propanedione. Camphorquinone is the most preferred alpha-diketone photoinitiator. According to a preferred embodiment, the polymerizable matrix contains the alpha-diketone sensitizer in an amount from 0.05 to 5 mole percent.

Preferably, in the compounds of formula (XIII), M is Si.

For example, compounds of formula (XIII) wherein $R^{11}$ has the formula (XVI) and which are symmetrical may be have the following structural formulae:

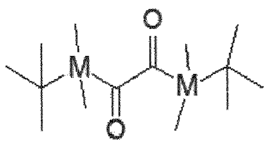

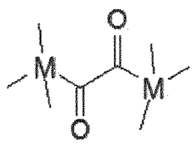

.

For example, compounds of formula (XIII) wherein $R^{11}$ represents a group of formula (XVI) wherein X is a bond, an oxygen atom or a $NR^{X1}$ group, and R* represents a substituted or unsubstituted hydrocarbyl group may have the following structural formulae:

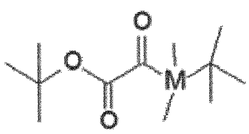

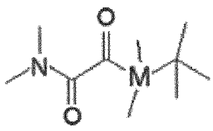

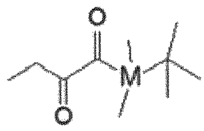

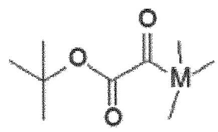

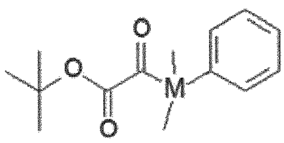

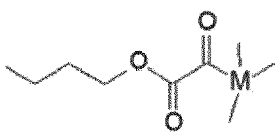

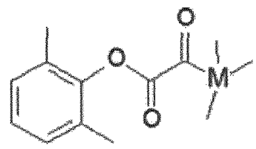

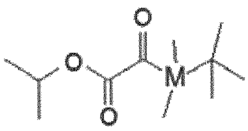

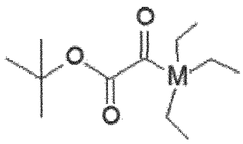

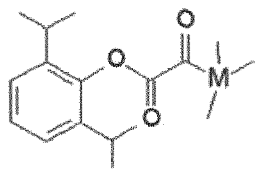

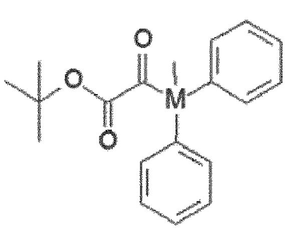

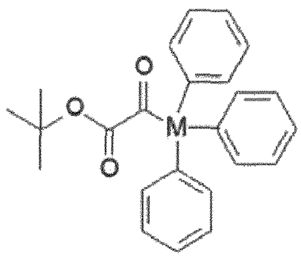

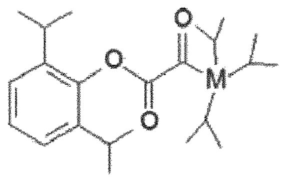 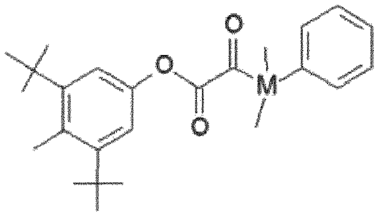

For example, compounds of formula (XIII) wherein R represents a group of formula (XVI) wherein R* represents a trihydrocarbylsilyl group have the following structural formulae:

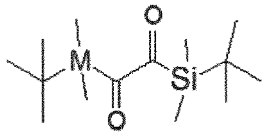 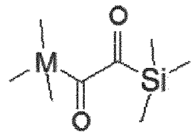.

For example, compounds of formula (XIII) wherein M is Si and $R^{11}$ represents a substituted or unsubstituted hydrocarbyl group may have the following structural formulae:

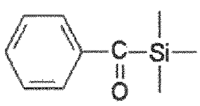 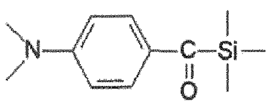 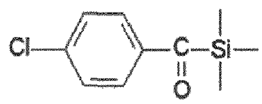

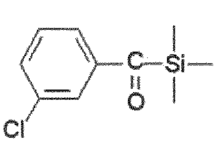 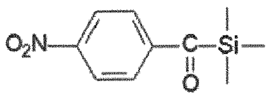 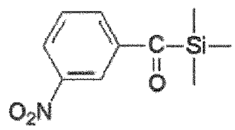

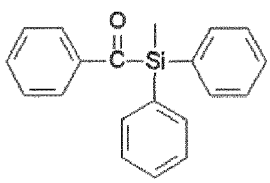 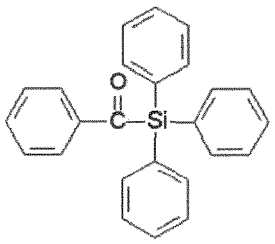 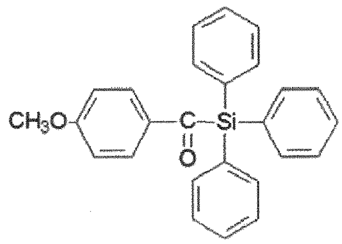

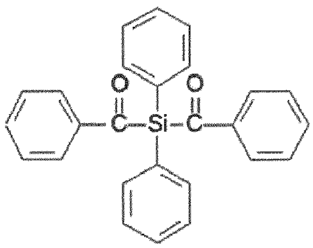 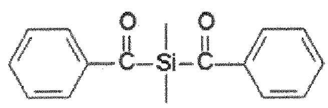 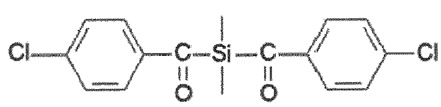

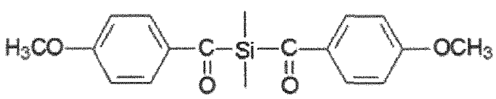 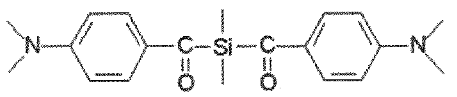

Preferably, compound of formula (XIII) is selected from the group consisting of:

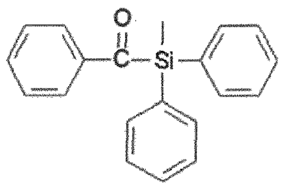 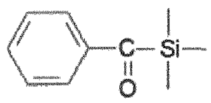 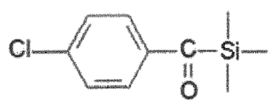 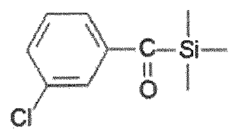

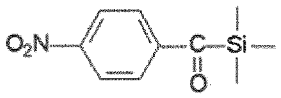 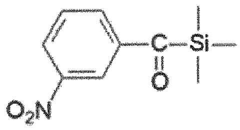 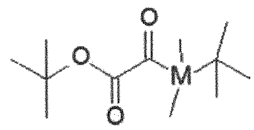 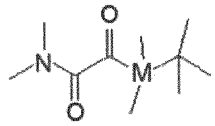

,

wherein compounds of formula (XIII) with M = Si are particularly preferred.

More preferably, compound of formula (XIII) is selected from the group consisting of:

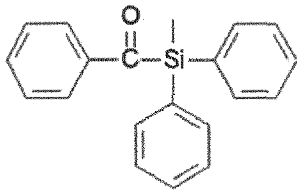 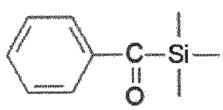 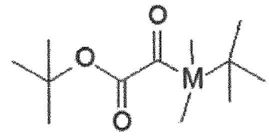

,

wherein it is particularly preferred that M = Si.

Most preferably, compound of formula (XIII) is tert-butyl (tert-butyldimethylsilyl)glyoxylate) (DKSi).

A suitable photoinitiator system may also include phosphine oxides typically having a functional wavelength range of about 380 nm to about 1200 nm. Examples of phosphine oxide free radical initiators with a functional wavelength range of about 380 nm to about 450 nm include acyl and bisacyl phosphine oxides such as those described in US 4,298,738, US 4,324,744 US and 4,385,109 and EP 0 173 567. Specific examples of the acylphosphine oxides include 2,4,6-trimethylbenzoyldiphenylphosphine oxide, bis(2,4,6-trimethylbenzoyl)phenylphosphine oxide, dibenzoylphenylphosphine oxide, bis(2,6-dimethoxybenzoyl)phenylphosphine oxide, tris(2,4-dimethylbenzoyl)phosphine oxide, tris(2-methoxybenzoyl)phosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, benzoyl-bis(2,6-dimethylphenyl)phosphonate, and 2,4,6-trimethylbenzoylethoxyphenylphosphine oxide. Commercially available phosphine oxide photoinitiators capable of free-radical initiation when irradiated at wavelength ranges of greater than about 380 nm to about 450 nm include bis(2,4,6-trimethylbenzoyl)phenyl phosphine oxide (IRGACURE 819), bis(2,6-dimethoxybenzoyl)-(2,4,4-trimethylpentyl) phosphine oxide (CGI 403), a 25:75 mixture, by weight, of bis(2,6-dimethoxybenzoyl)-2,4,4-trimethylpentyl phosphine oxide and 2-hydroxy-2-methyl-1-phenylpropan-1-one (IRGACURE 1700), a 1:1 mixture, by weight, of bis(2,4,6-trimethylbenzoyl)phenyl phosphine oxide and 2-hydroxy-2-methyl-1-phenylpropane-1-one (DAROCUR 4265), and ethyl 2,4,6-trimethylbenzylphenyl phosphinate (LUCIRIN LR8893X). Typically, the phosphine oxide initiator is present in the composition in catalytically effective amounts, such as from 0.1 percent by weight to 5.0 percent by weight, based on the total weight of the composition.

Suitable electron donor compounds include substituted amines, e.g., ethyl dimethylaminobenzoate or dimethylamino benzonitrile.

Tertiary amine reducing agents may be used in combination with an acylphosphine oxide Examples of suitable aromatic tertiary amine include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, 4-N,N-dimethylaminobenzoic acid ethyl ester, 4-N,N-dimethylaminobenzoic acid methyl ester, 4-N,N-dimethylaminobenzoic acid n-butoxyethyl ester, 4-N,N-dimethylaminobenzoic acid 2-(methacryloyloxy) ethyl ester, 4-N,N-dimethylaminobenzophenone ethyl 4-(N,N-dimethylamino)benzoate and N,N-dimethylaminoethyl methacrylate. Examples of an aliphatic tertiary amine include trimethylamine, triethylamine, N-methyldiethanolamine, N-ethyldiethanolamine, N-n-butyldiethanolamine, N-lauryldiethanolamine, triethanolamine, 2-(dimethylamino) ethyl methacrylate, N-methyldiethanolamine dimethacrylate, N-ethyldiethanolamine dimethacrylate, triethanolamine monomethacrylate, triethanolamine dimethacrylate, and triethanolamine trimethacrylate.

The amine reducing agent may be present in the composition in an amount from 0.1 percent by weight to 5.0 percent by weight, based on the total weight of the composition.

According to a futher preferred embodiment, the photo initiator further comprises an iodonium compound of the following formula (XVII):

$$R^{12}\text{-}I^{+}\text{-}R^{13}A^{-} \qquad \text{(XVII)}$$

wherein

$R^{12}$ and $R^{13}$
which are independent from each other, represent an organic moiety, and $A^-$ is an anion;

For example, diaryl iodonium salt may be selected from (4-methylphenyl)[4-(2-methylpropyl) phenyl] iodonium hexafluoroantimonate, include (4-methylphenyl)[4-(2-methylpropyl) phenyl] iodonium tetrafluoroborate, diphenyliodonium (DPI) tetrafluoroborate, di(4-methylphenyl)iodonium (Me2-DPI) tetrafluoroborate, phenyl-4-methylphenyliodonium tetrafluoroborate, di(4-heptylphenyl)iodonium tetrafluoroborate, di(3-nitrophenyl)iodonium hexafluorophosphate, di(4-chlorophenyl)iodonium hexafluorophosphate, di(naphthyl)iodonium tetrafluoroborate, di(4-trifluoromethylphenyl)iodonium tetrafluoroborate, DPI hexafluorophosphate, Me2-DPI hexafluorophosphate; DPI hexafluoroarsenate, di(4-phenoxyphenyl)iodonium tetrafluoroborat, phenyl-2-thienyliodonium hexafluorophosphate, 3,5-dimethylpyrazolyl-4-phenyliodonium hexafluorophosphate, DPI hexafluoroantimonate, 2,2'-DPI tetrafluoroborate, di(2,4-dichlorophenyl)iodonium hexafluorophosphate, di(4-bromophenyl)iodonium hexafluorophosphate, di(4-methoxyphenyl)iodonium hexafluorophosphate, di(3-carboxyphenyl)iodonium hexafluorophosphate, di(3-methoxycarbonylphenyl)iodonium hexafluorophosphate, di(3-methoxysulfonylphenyl)iodonium hexafluorophosphate, di(4-acetamidophenyl)iodonium hexafluorophosphate, di(2-benzothienyl)iodonium hexafluorophosphate, and DPI hexafluorophosphate.

Particularly preferred iodonium compounds include diphenyliodonium (DPI) hexafluorophosphate, di(4-methylphenyl)iodonium (Me2-DPI) hexafluorophosphate, diaryliodonium hexafluoroantimonate, (4-methylphenyl)[4-(2-methylpropyl) phenyl] iodonium hexafluoroantimonate, (4-methylphenyl)[4-(2-methylpropyl)phenyl]iodonium hexafluorophosphate (Irgacure® 250, commercial product available from BASF SE), (4-methylphenyl)[4-(2-methylpropyl) phenyl] iodonium tetrafluoroborate, 4-octyloxyphenyl phenyliodonium hexafluoroantimonate, 4-(2-hydroxytetradecyloxyphenyl)phenyliodonium hexafluoroantimonate, and 4-isopropyl-4'-methyldiphenyliodonium borate.

According to a particularly preferred embodiment, the iodonium compound is DPI hexafluorophosphate and/or 4-isopropyl-4'-methyldiphenyliodonium tetrakis(pentafluorophenyl) borate.

According to a futher preferred embodiment, the photo initiator further comprises a sulfonium compound of the following formula (XIV):

$$R^{14}R^{15}R^{16}S^{+}A^{-} \qquad \text{(XIV)}$$

wherein

$R^{14}$, $R^{15}$ and $R^{16}$
which are independent from each other, represent an organic moiety or wherein any two of $R^{14}$, $R^{15}$ and $R^{16}$ form a cyclic structure together with the sulfur atom to which they are bound, and
$A^-$ is an anion.

A preferred triaryl sulfonium salt is S-(phenyl)thianthrenium hexafluorophosphate:

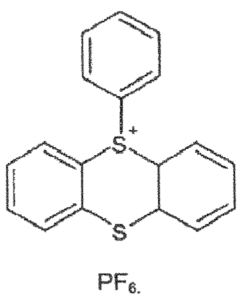

$PF_6$.

According to a futher preferred embodiment, the photo initiator further comprises a phosphonium compound of the following formula (XV):

$$R^{17}R^{18}R^{19}R^{20}P^{+}A^{-} \qquad (XV)$$

wherein

$R^{17}$, $R^{18}$, $R^{19}$, and $R^{20}$
which are independent from each other, represent an organic moiety, and $A^{-}$ is an anion.

Particularly preferred phosphonium salts are the tetraalkyl phosphonium salts tetrakis-(hydroxymethyl)-phosphonium (THP) salt or a tetrakis-(hydroxymethyl)-phosphonium hydroxide (THPOH) salt, wherein the anion of the tetraalkyl phosphonium salt is selected from the group consisting of formate, acetate, phosphate, sulphate, fluoride, chloride, bromide and iodide.

According to a preferred embodiment, the light-curable dental impression material comprises 0.1 to 5 percent by weight, more preferably 1 to 4 percent by weight, based on the total weight of the light-curable dental impression material, of a photoinitiator.

<u>Optional polymerizable (meth)acrylates or (meth)acrvlamides</u>

The light-curable dental impression material of the present invention may further comprise with up to 20 percent by weight based on the total weight of the composition of polymerizable (meth)acrylates or (meth)acrylamides.

The (meth)acrylate compounds may be selected from methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate (HEMA), hydroxypropyl acrylate, hydroxypropyl methacrylate, tetrahydrofurfuryl acrylate, tetrahydrofurfuryl methacrylate, glycidyl acrylate, glycidyl methacrylate, bisphenol A glycerolate dimethacrylat ("bis-GMA", CAS-No. 1565-94-2), 4,4,6,16 (or 4,6,6,16)-tetramethyl-10,15-dioxo-11,14-dioxa-2,9-diazaheptadec-16-enoicacid 2-[(2-methyl-1-oxo-2-propen-1-yl)oxy]ethyl ester (CAS no. 72869-86-4)_(UDMA), glycerol mono-and di- acrylate such as 1,3-glycerol dimethacrylate (GDM), glycerol mono-and dimethacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyt-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane,and 2,2'-bis[3(4-phenoxy)-2-hydroxypropane-1-acrylate]propane.

Preferred (meth)acrylamides may be selected from the following compounds.

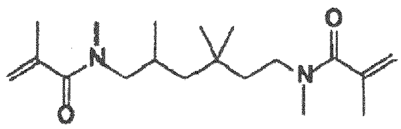

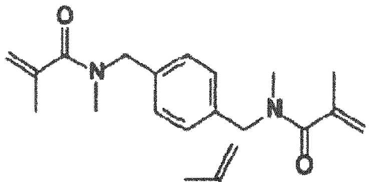

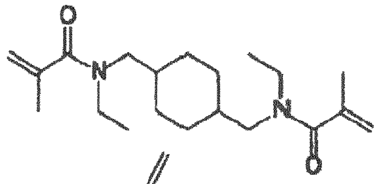

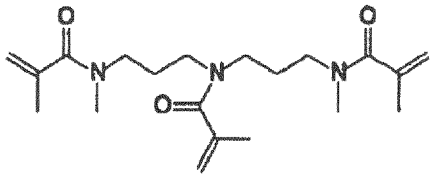

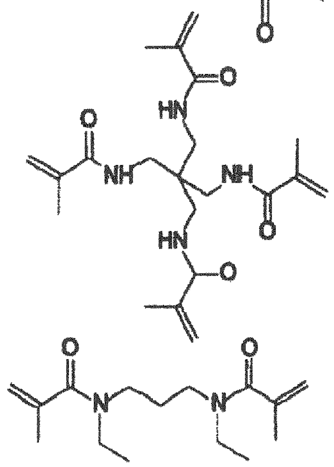

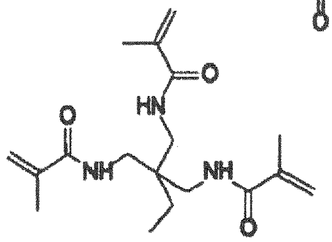

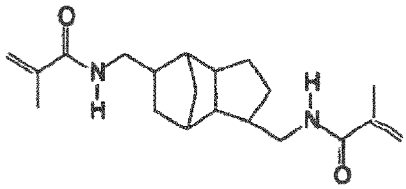

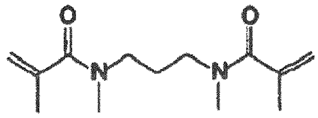

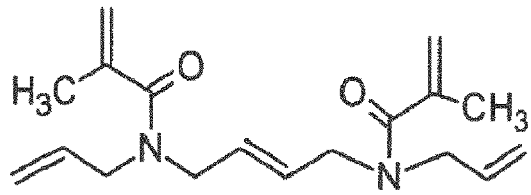

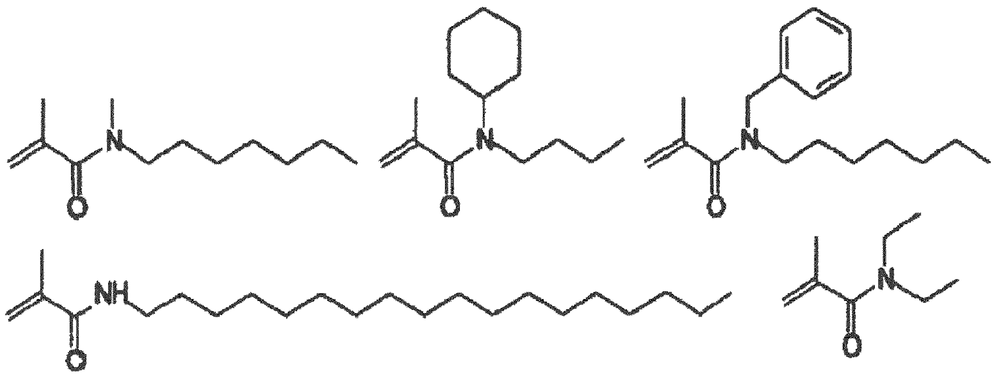

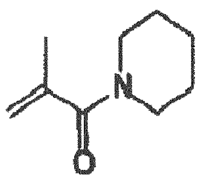

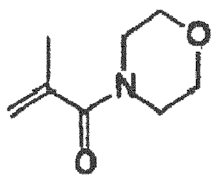

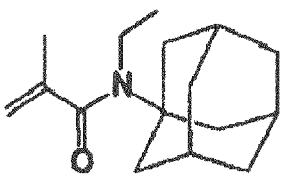

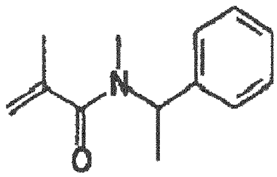

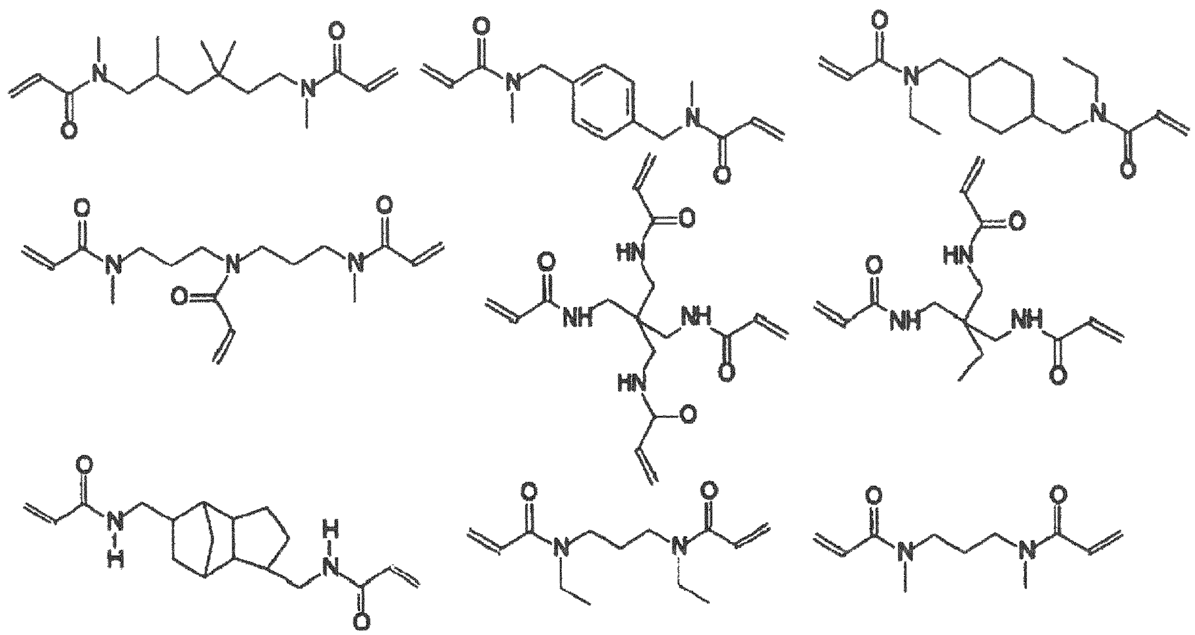

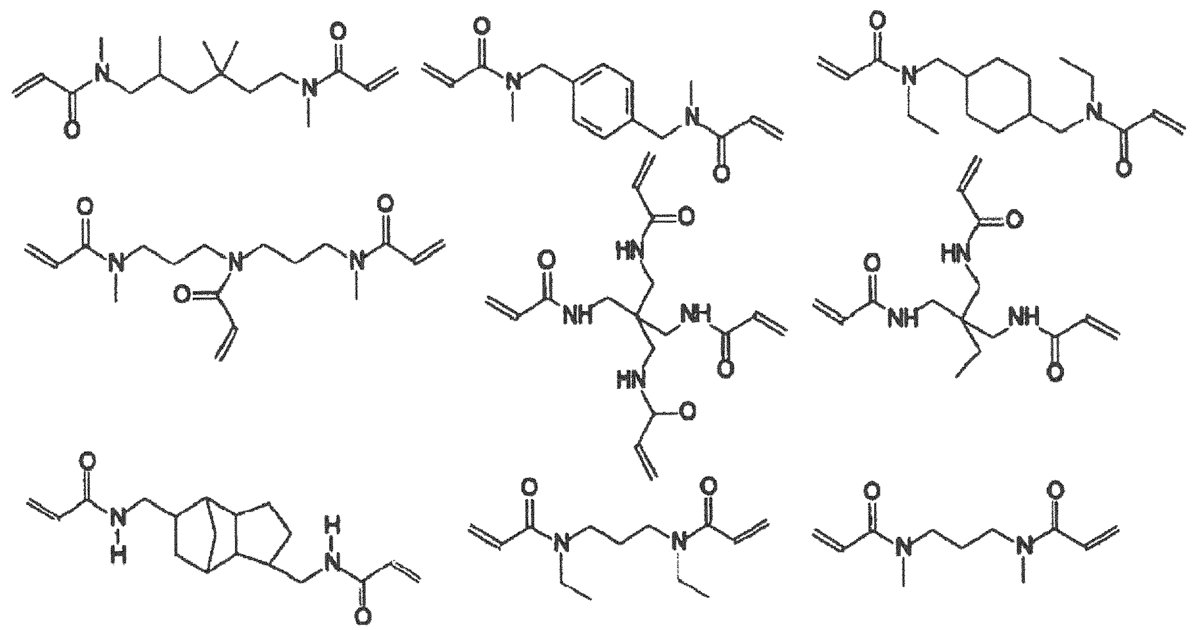

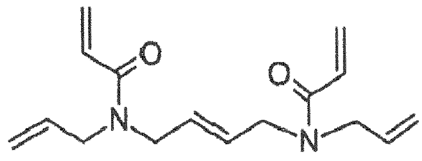

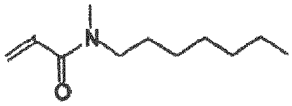
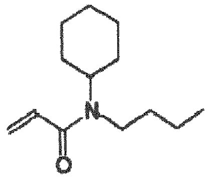
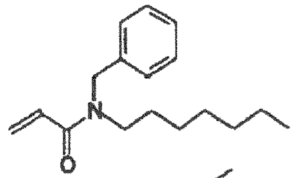
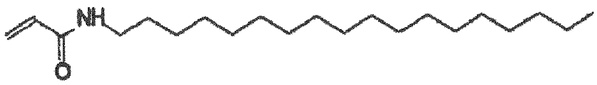
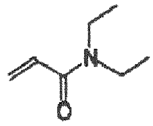
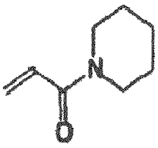
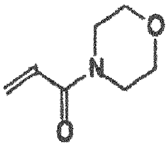
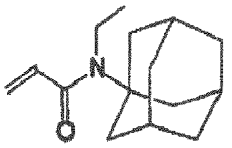
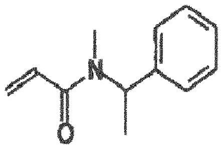

Most preferred are the bis-(meth)acrylamides:

N,N'-diallyl-1,4- bisacrylamido-(2E)-but-2-en (BAABE) having the structural formula

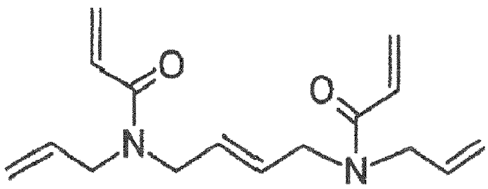
,

and

N,N'-diethyl-1,3-bisacrylamido-propan (BADEP) having the structural formula

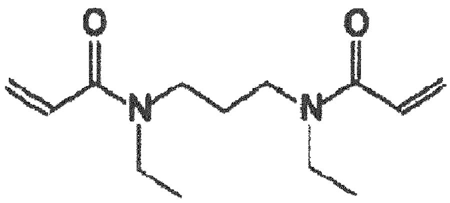

Further components

Optionally, the light-curable dental impression material of the present invention may further comprise stabilizer(s), and/or pigments. Moreover, the light-curable dental impression material of the present invention may further comprise cationically polymerizable monomers.

The term "stabilizer" as used herein means any compound capable of preventing polymerizable compounds contained in the light-curable dental impression material from spontaneous polymerization during storage. However, the stabilizer does not disturb or prevent intended polymerisation curing of the light-curable dental impression material during application.

For example, the stabilizer may be a conventional stabilizer selected from the group consisting of hydroquinone, hydroquinone monomethylether, tert-butylhydroquinone, tert-butylhydroxyanisol, propyl gallate and 2,6-di-tert-butyl-p-cresol. From these conventional stabilizers, 2,6-di-tert-butyl-p-cresol is preferred. Furthermore, anaerobic stabilzers such TEMPO, phenothiazine, galvanoxyl radical may be used.

The light-curable dental impression material according to the invention may contain the stabilizer in an amount of 0.001 to 1 percent by weight, preferably 0.005 to 0.8 percent by weight based on the total weight of the composition. When the amount of the stabilizer is below the above indicated lower limit of 0.001, then storage stability of the light-curable dental impression material might be insufficient, since the amount of stabilizer is too small to provide a stabilizing

effect. However, when the amount of stabilizer is above the maximum threshold of 1 percent by weight, then the applicability of the light-curable dental impression material might be negatively affected, since higher amounts of stabilizer may disturb or even substantially prevent intended polymerization curing of the light-curable dental impression material during application.

According to a specific embodiment, the light-curable dental impression material according to the invention may include additional cationic polymerizable resins including a compound having one or more cationic polymerizable groups according to the following formula (XVI).

$$\left(K\right)_{o}R^{21} \quad \text{(XVI)}$$

wherein

K= cationically polymerizable group
$R^{21}$= organic moiety
o≥ 1

Preferably, K represents a vinyl ether group, a vinyl ester group, a vinyl siloxane group, an epoxide group, an oxetane group and a furane group.

More preferably, K represents a vinyl ether group and a vinyl ester group, most preferably K represents a vinyl ether group.

Preferably, $R^{21}$ represents an o-valent $C_{1-30}$ hydrocarbyl groups which may contain 1-15 heteroatoms selected from O, S, Si, and which may be substituted by 1-15 substituents selected from $C_{1-4}$ alkyl groups, $C_{4-10}$ aryl groups, $C_{4-9}$ heteroaryl groups, halogen atoms, $C_{1-4}$ alkoxy groups, ester groups, thioether groups, silyl groups, and siloxane groups.

The hydrocarbyl group may be an alkyl group, a cycloalkyl group, a cycloalkylalkyl group, an arylalkyl group or an aryl group.

An alkyl group may be straight-chain or branched $C_{1-30}$ alkyl group, typically a $C_{1-6}$ alkyl group. Examples for a $C_{1-6}$ alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl.

A cycloalkyl group may be a $C_{3-20}$ cycloalkyl group, typically a $C_{3-8}$ cycloalkyl group. Examples of the cycloalkyl group can include those having 3 to 6 carbon atoms, for example, cyclopropyl, cyclobutyl, cyclopentyl and cyclohexyl.

A cycloalkylalkyl group may have 4 to 20 carbon atoms and may include a combination of a linear or branched alkyl group having 1 to 6 carbon atoms and a cycloalkyl group having 3 to 14 carbon atoms. Examples of the cycloalkylalkyl group can for example, include methylcyclopropyl, methylcyclobutyl, methylcyclopentyl, methylcyclohexyl, ethylcyclopropyl, ethylcyclobutyl, ethylcyclopentyl, ethylcyclohexyl, propylcyclopropyl, propylcyclobutyl, propylcyclopentyl, propylcyclohexyl.

An arylalkyl group may be a $C_{7-20}$ arylalkyl group, typically a combination of a linear or branched alkyl group having 1 to 6 carbon atoms and an aryl group having 6 to 10 carbon atoms. Specific examples of an arylalkyl group are a benzyl group or a phenylethyl group.

An aryl group can include aryl groups having 6 to 10 carbon atoms. Examples of the aryl group are phenyl and naphtyl.

The hydrocarbylcarbonyl groups of $R^{21}$ represent acyl groups ($R^{22}$-(C=O)-) in which the organic residue $R^{22}$ is a hydrocarbyl residue as defined above.

Preferably, the hydrocarbylcarbonyl group is an arylcarbonyl group, more preferably a benzoyl group.

Preferably, $R^{21}$ is selected from the group consisting of a straight chain or branched $C_{1-6}$ alkyl group, and a phenyl or benzoyl group which may optionally be substituted by one to three substitutents selected from halogen atoms, $C_{1-4}$ alkoxy groups.

Preferably, o is between 1 and 4, more preferably o is 2.

More preferably, the reactive diluent is a compound of the formula (XVII)

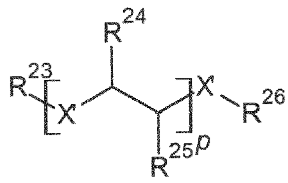

(XVII)

wherein

$R^{23}$ and $R^{26}$ which may be the same or different, independently represent a hydrogen atom, a $C_{1-4}$ alkyl group, a vinyl group, a vinyl silyl group, an epoxide group, an oxetane group, a furane group,
$R^{24}$ and $R^{25}$ which may be the same or different, independently represent a hydrogen atom, a $C_{1-4}$ alkyl group, or a vinyl ether group, a vinyl ester group, a vinyl siloxane group, an epoxide group, an oxetane group, a furane group,
X' represents an oxygen, a sulfur or a carbon atom,
p represents an integer of from 1 to 10,

provided that at least one cationic polymerizable group is present in the compound of formula (XVII).

In a preferable embodiment, X' represents an oxygen atom, $R^{24}$ represents a hydrogen atom, or a methyl group, $R^{25}$ represents a hydrogen atom, $R^{23}$ and $R^{26}$ represent vinyl groups, more preferable X' represents an oxygen atom, $R^{24}$ and $R^{25}$ represent a hydrogen atom, $R^{23}$ and $R^{26}$ represent vinyl groups.

A particular suitable reactive diluent is ethylene glycol vinyl ether.

The present invention also provides the use of the light-curable dental impression material according to the present invention for the preparation of a dental impression.

The present invention will now be further illustrated based on the following examples.

**Examples**

Typical compositions and processes to siloxane-modified Genesis Resin (Fig. 1) and PEGMA-modified Genesis Resin :

Preparation Example 1

Siloxane-modified Genesis Resin (Fig. 1):

The following raw materials were used.

| Raw Material | Formula | $M_n$ /g/mol |
|---|---|---|
| Poly(ethylene glycol) methacrylate | 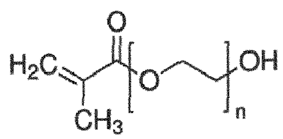 | ~ 360<br>~ 500 |
| TMDI | 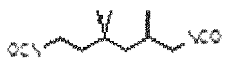 | 210 |
| Voranol 2120 | 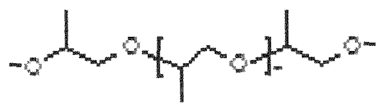 | 2000<br>4000 |
| Hydroxyl-terminated Siloxane (Carbinol 1K) | 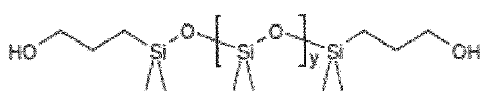 | 1000 |

(continued)

| Raw Material | Formula | $M_n$ /g/mol |
|---|---|---|
| bismuth 2-ethyl hexanoate K-KAT 348 | 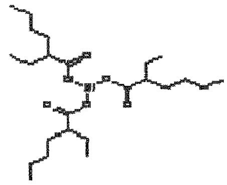 | |

Into a 1000ml resin kettle, the jacked temperature is set at 50°C and dry air is purged through the reactor. Then 121.7g (0.58mol) of trimethyl hexamethylene diisocyanate (TMDI), Then 466.8g (0.12mol) of Voranol 220-028, 284.0g (0.29mol) of Silmer monomer and 0.31g of urethane catalyst (K-Kat®348, a Bismuth Carboxylate from King Industries) were added into the reaction system and keep reaction for additional 2-3 hours. Then, a sample for FTIR and NMR was taken. As all hydroxyl groups were consumed, 116.6g (0.32mol) of PEGMA were added slowly to the reaction system for 30min. After 2hr reaction, 118.2g (0.10mol) of 1-dodecanol was added slowly into the system. The reaction was kept at 50°C overnight, then 0.4g of BHT was added to the system prior to discharge. Sampling was made for FTIR and NMR. The resin was placed in 45°C to clear out all the trapped air bubbles. Viscosity of 11Pa.s @25°C resulted with MA content as 0.32mmol/g and siloxane content as 28.2% wt/wt.

Examples 2 to 14

In a similar manner as described in Preparation Example 1, further siloxane-modified polyether-urethane dimethacrylate macromonomers were prepared.

The composition and properties of macromonomers of Preparative Examples 1 to 14 are as shown in the following Tables 1 and 2

**Table I: Compositions of Siloxane-modified Polyether-urethane Dimethacrylate Macromonomers**

| Examples | Batch Size (grams) | Reaction Sequence (stepwise) | Voranol 2K/4K (grams) (mole) | DMS-C16 DMS-C21 (grams) | TMDI (grams) | K-KAT348 (grams) (ppm) | PEGMA 400 (grams) (mole) | Viscosity @25°C (Pa.s) | MA Content mmol/g |
|---|---|---|---|---|---|---|---|---|---|
| 1 | 1213 | Voranol 2K/4k to TMDI | 300.0<br>0.1500<br>205.7<br>0.0514 | DMS-C21<br>450<br>0.100 | 105.45<br>0.5012 | 0.33<br>270 | 151.4<br>0.4205 | 34<br>opaque | 0.35 |
| 2 | 870 | Voranol 4k to TMDI | 401.80<br>0.1002 | DMS-C16<br>145.3<br>0.207 | 105.30<br>0.2010 | 0.30<br>340 | PE-350<br>193.3<br>0.537 | 14 Clear got gelled in 3wksRT | 0.58 |
| 3 | 1028 | Voranol 2K to TMDI | 500.8<br>0.2500 | DMS-C21<br>250<br>0.0500 | 105.60<br>0.5000 | 0.33<br>320 | 172.0<br>0.47 | 20<br>opaque | 0.46 |
| 4 | 816 | Voranol 4k to TMDI | 400.4<br>0.100 | DMS-C16<br>140.0<br>0.200 | 106.6<br>0.0501 | 0.33<br>340 | 170.0<br>0.47 | 12 slightly hazy | 0.58 |
| 5 | 1035 | Voranol 2K/4Lk to TMDI | 315.0<br>0.1575<br>360<br>0.090 | DMS-C21<br>113<br>0.0225 | 95.0<br>0.450 | 0.33<br>320 | 170.0<br>0.47 | 24<br>opaque | 0.41<br>Silox<br>10.9% |
| 6 | 695 | Voranol 2k/4k to TMDI | 120.0<br>0.060<br>160.0<br>0.040 | DMS-C16<br>140.0<br>0.200 | 105.5<br>0.050 | 0.33<br>340 | 170.0<br>0.47 | 12 slightly hazy | 0.68<br>Silox<br>20.1% |
| 7 | 796 | Voranol 4k to TMDI | 400.0<br>0.100 | DMS-C16<br>175.0<br>0.250 | 105.5<br>0.050 | 0.33<br>340 | 100.0<br>0.47<br>C12OH<br>0.08 | 14 slightly hazy | 0.35<br>Silox<br>22.0% |

**Table 2: Compositions of Siloxane-modified Polyether-urethane Dimethacrylate Macromonomers**

| Resin Code | Batch Size (grams) | Reaction Sequence (stepwise) | Voranol 2K (grams) (mole) | **Silmer OH Di-10** (grams) | TMDI (grams) | K-KAT348 (grams) (ppm) | PEGMA 400 (grams) (mole) | Viscosity @25°C (Pa.s) | MA Content mmol/g |
|---|---|---|---|---|---|---|---|---|---|
| 8 | 875 | Three-steps: V4k/HTSi/TMDI PEGMA C12OH | 400.1<br>0.100 | 255.0<br>0.250 | 105.56<br>0.50 | 0.33<br>340 | 100.0<br>0.278<br>15.0<br>0.08 | 12<br>clear | 0.318<br>Silox<br>20.0% |
| 9 | 890 C12OH-free | Two-steps: V4k/HTSi/TMDI PEGMA | 400.1<br>0.100 | 255.0<br>0.250 | 105.56<br>0.50 | 0.33<br>340 | *129.0*<br>*0.358* | *13*<br>*clear* | *0.402*<br>*Silox*<br>*28.6%* |
| 10 | 1003 | Three-steps: V4k/HTSi/TMDI PEGMA C12OH | 463.8<br>0.116 | 282.1<br>0.277 | 121.7<br>0.579 | 0.30<br>300 | 116.6<br>0.324<br>17.3<br>0.09 | 11<br>clear | 0.323<br>Silox<br>20.0% |
| 11 | 1007 | Three-steps: V4k/HTSi/TMDI PEGMA C12OH | 466.8<br>0.117 | 284.0<br>0.278 | 121.8<br>0.579 | 0.30<br>300 | 116.6<br>0.324<br>18.2<br>0.10 | 11<br>clear | 0.322<br>Silox<br>20.0% |
| 12 | 1006 Low Temp. | Three-steps: V4k/HTSi/TMDI PEGMA C12OH | 464.9<br>0.116 | 285.6<br>0.280 | 121.9<br>0.579 | 0.32<br>320 | 116.1<br>0.324<br>17.0<br>0.09 | 13<br>clear | 0.322<br>Silox<br>20.0% |
| 13 | 1006 Dosing | Three-steps: V4k/TMDI+HTSi PEGMA C12OH | 464.9<br>0.116 | 285.6<br>0.280 | 121.9<br>0.579 | 0.32<br>320 | 116.1<br>0.324<br>17.0<br>0.09 | 15<br>clear | 0.322<br>Silox<br>20.0% |
| 14 | 1006 Low Cat. | Three-steps: V4k/HTSi+TMDI PEGMA C12OH | 464.9<br>0.116 | 285.6<br>0.280 | 121.9<br>0.579 | 0.21<br>210 | 116.1<br>0.324<br>17.0<br>0.09 | 13<br>clear | 0.322<br>Silox<br>20.0% |

Example 15 (Reference)

PEGMA-modified Genesis Resin as additional polymerizable macromonomers (Fig. 2):

The following raw materials were used.

| Raw Material | Formula | $M_n$ / g/mol |
|---|---|---|
| Poly(ethylene glycol) methacrylate | 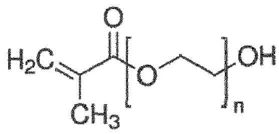 | ~ 360<br>~ 500 |
| TMDI | 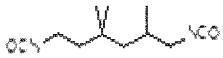 | 210 |
| Voranol 2120 | 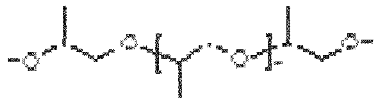 | 2000<br>4000 |
| bismuth 2-ethyl hexanoate K-KAT 348 | 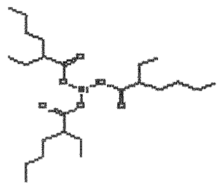 | |

Into a 1000ml resin kettle, the jacked temperature is set at 50°C and dry air is purged through the reactor. Then 105.35g (0.50mol) of trimethyl hexamethylene diisocyanate (TMDI), then 400.4g (0.20mol) of Voranol 220-056, 400.6g (0.10mol) of Voranol 220-028 and 0.304g of urethane catalyst (K-Kat®348, a Bismuth Carboxylate from King Industries) were added into the reaction system and the reaction is kept for additional 2-3 hours. Then samples for FTIR and NMR were taken. As all hydroxyl groups were consumed, 161.9g (0.45mol) of PEGMA was added slowly to the reaction system for 60min. The reaction was kept at 50°C overnight, then 0.4g of BHT was added to the system prior to discharge. Sampling was made for FTIR and NMR. The resin was placed in 45°C to clear out all the trapped air bubbles. Viscosity of 24Pa.s @25°C was resulted with MA content as 0.42mmol/g.

Reference Examples 16 to 39

In a similar manner as described in Reference Example 15, further PEGMA-modified polyether-urethane dimethacrylate macromonomers were prepared.

The composition and properties of macromonomers of Reference Examples 15 to 39 are as shown in the following Tables 3 and 4

**Table 3: Compositions of PEGMA-modified Polyether-urethane Dimethacrylate Oligomers**

| Examples | Batch Size (grams) | Reaction Sequence (stepwise) | Voranol 2K (grams) (mole) | TMDI (grams) | K-KAT348 (grams) (ppm) | PEGMA-400 (grams) (mole) | 4EG Diol (grams) (mole) | Viscosity @25°C (Pa.s) | MA Content mmol/g |
|---|---|---|---|---|---|---|---|---|---|
| 15 | 862 | Voranol 2K to TMDI | 545.95 0.2730 | 114.85 0.5462 | 0.1302 150 | 201.59 0.56 | | 13 clear | 0.65 |
| 16 | 810 | Voranol 2K to TMDI | 546.03 0.2730 | 114.93 0.5466 | 0.1305 160 | 129.73 0.3604 | 19.42 0.1000 | 35 clear | 0.44 |
| 17 | 814 | Voranol 2K to TMDI | 545.594 0.2730 | 114.80 0.5460 | 0.1341 160 | 134.61 0.374 | 19.41 0.1000 | 37 clear | 0.44 |
| 18 | 814 | Voranol 2K to TMDI | 545.76 0.2730 | 114.89 0.5464 | 0.2010 245 | 134.11 0.3725 | 19.50 0.1004 | 42 clear | 0.44 |
| 19 | 795 | Voranol 2K to TMDI | 545.74 0.2730 | 114.76 0.5457 | 0.1972 250 | 134.42 0.3734 | | 14 clear | 0.46 |
| 20 | 800 | Voranol 2K to TMDI | 546.71 0.2729 | 114.75 0.5457 | 0.3032 380 | 129.41 0.3595 | BDO 9.03 0.1002 | 64 clear | 0.45 |
| 21 | 800 | Voranol 2K to TMDI | 545.93 0.2730 | 114.73 0.5456 | 0.3025 380 | 215.72 0.5992 | | 11 slightly hazy/noodor | 0.68 |
| 22 | 878 | Voranol 2K to TMDI | 546.93 0.2730 | 114.84 0.5461 | 0.3022 340 | 129.81 0.3606 | BDO 8.99 0.0998 | 62 clear | 0.45 |
| 23 | 950 | Voranol 2K to TMDI | 660.02 0.3300 | 114.83 0.5460 | 0.2980 250 | 174.94 0.4859 | | 24 clear | 0.51 |
| 24 | 945 | Voranol 2K to TMDI | 660.2 0.3301 | 114.74 0.5455 | 0.3031 320 | PEM6 LD 169.94 0.486 | | 26 clear strong odor | 0.51 |
| 25 | 1053 | Voranol 2K to TMDI | 659.77 0.3296 | 114.79 0.5459 | 0.3112 300 | PEM63P 277.91 0.5151 | | 15 Clear/strong odor | 0.49 |

(continued)

| Examples | Batch Size (grams) | Reaction Sequence (stepwise) | Voranol 2K (grams) (mole) | TMDI (grams) | K-KAT348 (grams) (ppm) | PEGMA-400 (grams) (mole) | 4EG Diol (grams) (mole) | Viscosity @25°C (Pa.s) | MA Content mmol/g |
|---|---|---|---|---|---|---|---|---|---|
| 26 | 815 | Voranol 2K to TMDI | 545.89<br>0.2729 | 114.85<br>0.5462 | 0.3215<br>390 | PEGMA400<br>139.52<br>0.3876 | Isosorbide<br>14.59<br>0.0998 | 60<br>clear | 0.48 |
| 27 | 815 | Voranol 2K to TMDI | 546.12<br>0.2721 | 114.93<br>0.5466 | 0.3205<br>390 | PEGMA400<br>139.51<br>0.3875 | Isosorbide<br>14.60<br>0.0999 | 60<br>clear | 0.48 |
| 28 | 1012 | Voranol 2K to TMDI | 639.89<br>0.3199 | 134.78<br>0.6410 | 0.1609<br>160 | PEGMA400<br>237.16<br>0.6588 | | 13<br>clear | 0.65 |
| 29 | 1012 | Voranol 2K to TMDI | 639.75<br>0.3199 | 134.81<br>0.6411 | 0.1623<br>160 | PEGMA400<br>237.42<br>0.6595 | | 13<br>clear | 0.65 |
| 30 | 1005 | Voranol 2K to TMDI | 639.35<br>0.3197 | 134.56<br>0.6400 | 0.1661<br>165 | PEM6 LD<br>230.65<br>0.659 | | 12<br>clear strong odor | 0.66 |

**Table 4: Compositions of PEGMA-modified Polyether-urethane Dimethacrylate Oligomers**

| Examples | Batch Size (grams) | Reaction Sequence (stepwise) | Voranol 2K (grams) (mole) | TMDI (grams) | K-KAT348 (grams) (ppm) | PEGMAs (grams) (mole) | Chain Extnder (grams) (mole) | Viscosity @25°C (Pa.s) | MA Content mmol/g |
|---|---|---|---|---|---|---|---|---|---|
| 31 | 1012 | TMDI to Voranol 2K | 639.93<br>0.3200 | 134.68<br>0.6405 | 0.1652<br>160 | PEGMA400<br>237.55<br>0.6599 | | 25<br>clear | 0.65 |
| 32 | 1005 | All-in-One | 640.02<br>0.2730 | 134.66<br>0.5460 | 0.1641<br>160 | PEM6 LD<br>230.65<br>0.659 | | 7<br>clear<br>strong odor | 0.66 |
| 33 | 1012 | All-in-One | 640.13<br>0.3201 | 134.80<br>0.6411 | 0.1604<br>160 | PEGMA 400<br>237.03<br>0.6584 | | 6<br>clear | 0.65 |
| 34 | 970 | Voranol 4K to TMDI | 799.92<br>0.2000 | 63.13<br>0.3002 | 0.2611<br>270 | PE-350<br>107.05<br>0.2499 | | 19<br>clear | 0.26 |
| 35 | 930 | Voranol 4K to TMDI | 800.12<br>0.2000 | 57.85<br>0.2751 | 0.2539<br>270 | PEGMA 400<br>71.80<br>0.1994 | | 57<br>clear | 0.22 |
| 36 | 770 | Voranol 2K/4k to TMDI | 160.14<br>0.0801<br>479.99<br>0.1200 | 60.04<br>0.2855 | 0.2511<br>330 | PEGMA 400<br>68.44<br>0.1901 | | 60<br>clear | 0.25 |
| 37 | 770 | Voranol 2K/4k to TMDI | 159.99<br>0.080<br>480.18<br>0.1200 | 59.92<br>0.2850 | 0.2590<br>340 | PEGMA 400<br>68.63<br>0.1906 | | 59<br>clear | 0.25 |
| 38 | 1012 | Voranol 2K to TMDI | 639.83<br>0.3199 | 134.50<br>0.6396 | 0.1624<br>160 | PEGMA 400<br>237.73<br>0.6604 | | 12<br>clear | 0.65 |

(continued)

| Examples | Batch Size (grams) | Reaction Sequence (stepwise) | Voranol 2K (grams) (mole) | TMDI (grams) | K-KAT348 (grams) (ppm) | PEGMAs (grams) (mole) | Chain Extnder (grams) (mole) | Viscosity @25°C (Pa.s) | MA Content mmol/g |
|---|---|---|---|---|---|---|---|---|---|
| 39 | 930 | All-in-One 2-steps | 540.05<br>0.2700 | 134.67<br>0.6404 | 0.1673<br>180 | PEGMA 400<br>237.44<br>0.6596 | Monola<br>urin<br>13.64<br>0.0497 | 12<br>clear | 0.71 |

Examples 40 to 50

The effect of the molecular weight of carbinols on the appearance of the siloxane-modified macromonomers was investigated. The results are summarized in Table 5.

Table 5: Effect of Molecular Weight of Carbinols on Appearance of Siloxane-modified Genesis Resins

| Examples | Siloxane Segments | Voranol Segments | Siloxane Content %, *wt/wt* | MA Content mmol/g | Viscosity @25°C Pa.s | Appearance |
|---|---|---|---|---|---|---|
| 40 | DMS-C21 5000 | Voranol 2K Voranol 4K (1.5/1) | 37.3 | 0.35 | 34 | white opaque |
| 41 | DMS-C16, 600-850 | Voranol 4K | 16.7 | 0.54 | 14 | hazy and gelled |
| 42 | DMS-C21/ 5000 | Voranol 2K | 24.4 | 0.46 | 25 | white opaque |
| 43 | DMS-C16, 600-850 | Voranol 4K | 17.2 | 0.58 | 12 | slightly hazy |
| 44 | DMS-C21 5000 | Voranol 2K Voranol 4K (0.875/1) | 10.9 | 0.41 | 24 | opaque |
| 45 | DMS-C16, 600-850 | Voranol 2K Voranol 4K (0.75/1) | 20.1 | 0.68 | 12 | slightly hazy |
| 46 | DMS-C16, 600-850 | Voranol 4K | 22.0 | 0.35 | 14 | slightly hazy |
| 47 | DMS-C16, 600-850 | Voranol 4K | 14.5 | 0.29 | 16 | slightly hazy |
| 48 | DBE-C25 4000 | Voranol 2K | 8.4 | 0.43 | 22 | hazy gel-like |
| 49 | DBE-C25 4000 | Voranol 2K | 13.1 | 0.41 | 16 | hazy gel-like |
| 50 | none | Voranol 2K Voranol 4K (1/1) | 0 | 0.29 | 11 | clear |

Examples 51 to 53 (Reference)

Further additional polymerizable macromonomers in the form of trimethacrylated polyethers derived from Carpol triols were prepared as high molecular weight crosslinkers in accordance with the scheme shown in Fig. 3. The composition and properties of the additional polymerizable macromonomers are summarized in Table 6.

Table 6: Composition and Viscosity of *tri*Methacrylated Polyethers derived from Carpol Triols

| Examples | Carpol G1500 (grams) | Carpol G3000 (grams) | Carpol G5000 (grams) | ICEM (grams) | K-KAT 348 (grams) | Viscosity Pa.S@25°C | MA Content mmol/g |
|---|---|---|---|---|---|---|---|
| 51 | 0 | 0 | 765 | 153 | 0.35 | 24 | 1.17 |
| 52 | 0 | 612 | 0 | 204 | 0.35 | 62 | 0.73 |
| 53 | 550 | 0 | 0 | 368 | 0.35 | 101 | 0.49 |

Reference Examples 54 to 85 (Fig. 2, 4)

Standard Genesis resin with T-9 via TMDI to Voranol Process (T2V):

Into a 1000ml resin kettle, the jacked temperature is set at 50°C and dry air is purged through the reactor. Then 545.01 (0.2726mol) of Voranol-220-056 and 0.12g of urethane catalyst (T-9) was added. Then 114.22g (0.5431mol) of trimethyl hexamethylene diisocyanate (TMDI) was charged into an additional funnel and started to add slowing into the reaction system through 2 hours. Then took sample for FTIR and NMR. Then start to add 26.8g of HEMA to the reaction system in 30min, then 34.47g of 1,4-butanediol (BDO) was charged into the system in 30min too. After 1hr further reaction at 50°C, 65.01g of TIM preblend (ICEM) in 30min and it was left for further overnight reaction at 50°C, finally additional 13.7g of HEMA was added into the system. The reaction was kept in mixing at 50oC overnight. Then 0.4g of BHT was added to the system prior to discharge. Sampling was made for FTIR and NMR. The resin was placed in 45°C to clear out all the trapped air bubbles. Viscosity of 55Pa.s@25°C was resulted.

Standard Genesis resin with K-KAT via TMDI to Voranol Process (T2V):

Into a 1000ml resin kettle, the jacked temperature is set at 50°C and dry air is purged through the reactor. Then 546.27g (0.2732mol) of Voranol-220-056 and 0.14g of urethane catalyst (K-Kat®348, a bismuth carboxylate from King Industries) was added. Then 114.65g (0.5452mol) of trimethylhexamethylene diisocyanate (TMDI) was charged into an additional funnel and started to add slowing into the reaction system through 2 hours. Then took sample for FTIR and NMR. Then start to add 26.6g of HEMA to the reaction system in 30min, then 34.47g of 1,4 butanediol (BDO) was charged into the system in 30min too. After 1hr further reaction at 50 °C, 64.86g of TIM preblend (ICEM) in 30min and it was left for further overnight reaction at 50°C, finally additional 13.06g of HEMA was added into the system. The reaction was kept in mixing at 50°C overnight. Then 0.4g of BHT was added to the system prior to discharge. Sampling was made for FTIR and NMR. The resin was placed in 45°C to clear out all the trapped air bubbles. Viscosity of 120Pa.s @25°C was resulted.

Modified Genesis resin with K-KAT via Voranol to TMDI Process (V2T):

Into a 1000ml resin kettle, the Jacked temperature is set at 50oC and dry air is purged through the reactor. Then 154.73g (0.7358mol) of Trimethyl hexamethylene diisocyanate (TMDI) and 0.15g of urethane catalyst (K-Kat®348, a Bismuth Carboxylate from King Industries) was added. Then 545.77g (0.273mol) of Voranol-220-056 was charged into an additional funnel and started to add slowing into the reaction system through 2 hours. Then took sample for FTIR and NMR. Then start to add 39.04g (0.300mol) of HEMA to the reaction system in 30min, then 31.5 (0.35mol) of 1,4 butanediol (BDO) was charged into the system in 30min too. Keep the reaction at 50 °C overnight, then 0.4g of BHT was added to the system prior to discharge. Sampling was made for FTIR and NMR. The resin was placed in 45 °C to clear out all the trapped air bubbles. Viscosity of 90Pa.s @25 °C was resulted.

Modified Genesis resin with K-KAT via All-in One Process (All-in-One):

Into a 1000ml resin kettle, the jacked temperature is set at 50 °C and dry air is purged through the reactor. Then add 154.73g (0.7358mol) of trimethyl hexamethylene diisocyanate (TMDI), 545.95g (0.273mol) of Voranol-220-056, 64.71 g (0.497mol) of HEMA, 34.77 (0.3858mol) of 1,4 Butanediol (BDO) and 0.27g of urethane catalyst (K-Kat®348, a bismuth carboxylate from King Industries). The reaction was kept in mixing at 50 °C overnight, then 0.4g of BHT was added to the system prior to discharge. Sampling was made for FTIR and NMR. The resin was placed in 45 °C to clear out all the trapped air bubbles. Viscosity of 15Pa.s @25°C was resulted.

The composition and properties of the additional polymerizable macromonomers are summarized in Tables 7 to 10.

Table 7:

| Comparable Examples | Batch Size (grams) | Reaction Sequence (stepwise) | Voranol (grams) (mole) | TMDI (grams) | T-9 (grams) (ppm) | HEMA (grams) (mole) | 1,4-Butandiol (grams) (mole) | ICEM (grams) (mole) | Viscosity @25°C (Pa.s) |
|---|---|---|---|---|---|---|---|---|---|
| 54 | 800 | TMDI to Voranol | 545.31 (0.2726) | 114.91 (0.5465) | 0.11 (140) | 39.62 (0.3044) | 34.31 (0.3807)) | 64.92 (0.1905) | 35 |
| 55 | 800 | TMDI to Voranol | 545.01 (0.2725) | 114.22 (0.5431) | 0.12 (150) | 26.81+ 13.7 (0.3113) | 34.47 (0.3825) | 65.01 (0.1910) | 55 |
| 56 | 800 | TMDI to Voranol | 545.86 (0.2729) | 115.2 (0.5478) | 0.81 (1010) | 26.6+ 13.09 (0.3076) | 34.6 (0.3838) | 65.16 (0.1914) | 430 |
| 57 | 800 | TMDI to Voranol | 545.86 (0.2729) | 114.75 (0.5457) | 0.30 (370) | 26.7+ 13.2 (0.3061) | 34.7 (0.3852) | 65.0 (0.1909) | 70 |
| 58 | 800 | TMDI to Voranol | 544.85 (0.2701) | 114.74 (0.5457) | 0.12 (150) | 26.8+ 13.1 (0.3064) | 34.6 (0.3847) | 64.6 (0.1898) | 60 |
| 59 | 800 | TMDI to Voranol | 545.90 (0.2730) | 114.62 (0.5451) | 0.12 (150) | 26.7+ 13.1 (0.3058) | 34.4 (0.3825) | 64.7 (0.1900) | 60 |
| 60 | 800 | TMDI to Voranol | 546.05 (0.2725) | 114.68 (0.5455) | 0.12 (150) | 26.7+ 13.1 (0.3054) | 34.3 (0.3808) | 64.8 (0.1900 | 70 |
| 61 | 800 | TMDI to Voranol | 545.06 (0.2732) | 114.53 (0.5447) | 0.12 (150) | 26.6+ 13.1 (0.3047) | 34.6 (0.3835) | 64.2 (0.1887) | 75 |

Table 8:

| Reference Examples | Batch Size (grams) | Reaction Sequence (stepwise) | Voranol (grams) (mole) | TMDI (grams) | T-9 (grams) (ppm) | HEMA (grams) (mole) | 1,4-Butandiol (grams) (mole) | ICEM (grams) (mole) | Viscosity @25°C (Pa.s) |
|---|---|---|---|---|---|---|---|---|---|
| 62 | 800 | Voranol to TMD | 546.23 (0.2731) | 114.87 (0.5463) | 0.11 (140) | 26.97+13.4 (0.3102) | 34.59 (0.3838) | 68.2 (0.2003) | 20 |
| 63 | 200 | Voranol to TMD | 136.62 (0.0684) | 28.75 (0.1367) | 0.04 (200) | 6.62+3.31 (0.0762) | 8.64 (0.0959) | 16.205 (0.0476) | 10 |
| 64 | 200 | Voranol to TMD | 137.62 (0.0683) | 28.84 (0.1372) | 0.07 (350) | 9.62 (0.0739) | 8.72 (0.0968) | 16.5 (0.0485) | 15 |
| 65 | 200 | Voranol to TMD | 137.32 (0.0687) | 28.7 (0.1365) | 0.06 (300) | 6.72+3.57 (0.0791) | 8.98 (0.0996) | 16.34 (0.0480) | 10 |
| 66 | 800 | Voranol to TMD | 545.77 (0.2729) | 114.57 (0.5448) | 0.12 (150) | 26.8+13.27 (0.3079) | 34.48 (0.3826) | 65.02 (0.1910) | 10 |
| 67 | 800 | Voranol to TMD | 545.56 (0.2728) | 114.65 (0.5452) | 0.21 (260) | 39.88 (0.3064) | 34.86 (0.3868) | 66.41 (0.1951) | 35 |
| 68 | 800 | Voranol to TMD | 546.14 (0.2731) | 114.8 (0.5459) | 0.30 (380) | 26.7+13.16 (0.3060) | 34.65 (0.3845) | 65.12 (0.1913) | 40 |

Table 9:

| | Batch Size (grams) | Reaction Sequence (stepwise) | Voranol (grams) (mole) | TMDI (grams) | K-KAT348 (grams) (ppm) | HEMA (grams) (mole) | 1,4-Butandiol (grams) (mole) | ICEM (grams) (mole) | Viscosity @25°C (Pa.s) |
|---|---|---|---|---|---|---|---|---|---|
| 69 | 800 | All in **One-step** | 545.96 (0.2730) | 154.73 (0.7358) | **0.2743 (340)** | 64.71 (0.4972) | 34.77 (0.3858) | No ICEM | **15** |
| 70 | 800 | **TMDI to Voranol** | 546.30 (0.2732) | 114.8 (0.5459) | **0.2723 (340)** | 26.6+ 13.17 (0.3059) | 34.33 (0.3809) | 64.84 (0.1905) | **140** |
| 71 | 800 | **TMDI to Voranol** | 546.27 (0.2731) | 114.65 (0.5452) | **0.1393 (170)** | 26.6+ 13.13 (0.3057) | 34.47 (0.3825) | 64.86 (0.1905) | **120** |
| 72 | 800 | **Voranol to TMDI** | 546.4 (0.2732) | 114.57 (0.5448) | **0.1318 (170)** | 26.6+ 13.06 (0.3048) | 34.44 (0.3822) | 64.51 (0.1895) | **50** |
| 73 | 800 | **TMDI to Voranol** | 546.26 (0.2731) | 114.55 (0.5447) | **0.0646 (80)** | 26.7+ 13.09 (0.3054) | 34.32 (0.3808) | 64.56 (0.1896) | **45** |
| 74 | 800 | **Two-steps** | 546.09 (0.2730) | 114.53 (0.5447) | **0.0629 (80)** | 39.64 (0.3046) | 34.55 (0.3834) | 64.73 (0.1901) | **35** |
| 75 | 786 | **Voranol to TMDI** | 546.04 (0.2730) | 114.72 (0.5455) | **0.1350 (170)** | **26.30 (0.2021)** | 34.36 (0.3812) | 64.74 (0.1903) | **70** |
| **DABCO: 1,4-Diazabicyclo [2.2.2] octane** | | | | | | | | | |
| 76 | 800 | **TMDI to Voranol** | 546.14 (0.2731) | 114.63 (0.5451) | **0.4943+ 0.4959 (1240)** | 26.8+ 13.10 (0.3067) | 34.30 (0.3806) | 64.64 (0.1899) | **10** |

Table 10:

| Comparable Examples | Batch Size (grams) | Reaction Sequence (stepwise) | Voranol (grams) (mole) | TMDI (grams) | K-KAT348 (grams) (ppm) | HEMA (grams) (mole) | 1,4-Butandiol (grams) (mole) | ICEM (grams) (mole) | Viscosity @25°C (Pa.s) |
|---|---|---|---|---|---|---|---|---|---|
| 77 | 800 | **TMDI to Voranol** | 546.22<br>0.2731 | 114.85<br>0.5462 | 0.1316<br>**170** | 26.27<br>0.2019 | 34.32<br>0.3808 | 64.57<br>0.1897 | **80** |
| 78 | 800 | **Voranol to TMDI** | 545.71<br>0.2729 | **154.95**<br>0.7369 | 0.1360<br>**170** | 26.20<br>**26.22**<br>0.4028 | 34.45<br>0.3823 | No | **135** |
| 79 | 800 | **Voranol to TMDI** | 545.54<br>0.2723 | **154.90**<br>0.7366 | 0.1394<br>**170** | 26.17<br>**46.90**<br>0.5615 | **27.08**<br>0.3005 | No | **95** |
| 80 | 800 | **Voranol to TMDI** | 545.70<br>0.2730 | **154.89**<br>0.7366 | 0.1377<br>**170** | 39.02<br>0.2998 | 34.42<br>0.3819 | No | **90** |
| 81 | 800 | **Voranol to TMDI** | 545.98<br>0.2730 | **154.71**<br>0.7357 | 0.1377<br>**170** | 39.05<br>0.3001 | **31.57**<br>0.3503 | No | **110** |
| 82 | 800 | **Voranol to TMDI** | 545.77<br>0.2729 | **154.88**<br>0.7365 | 0.1372<br>**170** | 39.06<br>0.3001 | 34.41<br>0.388 | No | **90** |
| 83 | 800 | All in **1-step** | 546.05<br>0.2720 | **154.93**<br>0.7368 | **0.1411**<br>**200** | 64.63<br>0.4966 | 34.79<br>0.3860 | No<br>ICEM | **13**<br>**13** |
| 84 | 800 | **V2T+ HEMA-DBO 2-step** | 545.79<br>0.2729 | **154.96**<br>0.7369 | **0.1336**<br>**170** | 39.04<br>0.3000 | 34.72<br>0.3853 | No<br>ICEM | **130** |
| 85 | 800 | **IV2T+ HEMA-DBO 3-step** | 545.79<br>0.2729 | **154.96**<br>0.7369 | **0.1336**<br>**170** | *39.04*<br>*0.3000* | 34.72<br>0.3853 | 65.08<br>0.1912 | **30**<br>**80** |

**Example 86**

Combo Resins with 90/10 or 80/20 of Resin A comprising compounds of the formula (I) without any polysiloxane group and Resin B comprising compounds of the formula (I) according to the present invention, were also prepared by mixing all components of Resin A and Resin B in feed compositions (see Table I) as part of composition approach to further reduce the reaction rate involved in hydroxyl-terminated siloxane monomer. Indeed moderate temperature raisings of 5°C in 80/20 combo resin (ZZ2-4) and 9°C in 90/10 combo resin (ZZ1-208) in comparison to 12°C in Resin B only were found in such direct synthesis of Resin A and Resin B in 90/10 or 80/20 compositions.

Further, such Combo Resins were formulated (see Table II) and pastes were made (Table III) and evaluated (Table IV), accordingly. The compositions of corresponding activated resins (AL02-170-2) and KR2-11 and the compositions of formulated pastes thereof (AL02-171 and AL02-172, respectively, are shown below. Although paste from 90/10 Combo Resin (ZZ2-208) could offer excellent tear strength, it got lower Shore A hardness in comparison to the paste based on physically blended Resin A and Resin B in same composition, AL02-171 vs AL02-132 in Table IV.

**Table 11: Feed Compositions of Combo Resins**

| Resin | TMDI (mole ) | Voranol 4K (mole) | Voran ol 2K (mole) | Silme r (mole ) | K-KA T pp m | PEGM A 400 (mole) | C12O H (mole) | Viscosit y@25°C Pa.s | Si Conten t %wt/wt | MA Content Mmol/g |
|---|---|---|---|---|---|---|---|---|---|---|
| **Resin A (ZZ1-182)** | 4.69 | 0.94 | 1.87 | 0 | 285 | 4.22 | 0 | 23 | 0 | 0.42 |
| **Resin B (ZZ1-181)** | 0.50 | 0.10 | 0 | 0.25 | 340 | 0.28 | 0.08 | 12 | 29.1 | 0.32 |
| **Comb o Resin 90/10 (ZZ1-208)** | 0.48 | 0.10 | 0.17 | 0.03 | 325 | 0.42 | 0.01 | 22 | 3.0 | 0.41 |
| **Comb o Resin 80/20 (ZZ2-4)** | 0.49 | 0.10 | 0.15 | 0.06 | 210 | 0.40 | 0.02 | 20 | 5.65 | 0.40 |

**Table 12: Compositions of Activated Combo Resins**

| | AL02-170-2 | L171211 |
|---|---|---|
| **Activated Combo Resin with Resin A and Resin B in 90/10** | **Dual Initiator Formula %** | **Actual gms** |
| **ZZ1-208** | 98.875 | 197.74 |
| CQ | 0.125 | 0.25 |
| Recrystl. EDAB | 0.700 | 1.4 |
| Omnirad 380 | 0.250 | 0.50067 |
| BHT | 0.050 | 0.10947 |
| **Total** | **100.00** | **200** |
| | | |
| | **KR2-11** | **L171212** |
| **Activated Combo Resin with Resin A and Resin B in 90/10** | **Dual Initiator Formula %** | **Actual gms** |
| **ZZ2-4** | 98.875 | 197.75 |
| CQ | 0.125 | 0.25 |
| Recrystl. EDAB | 0.700 | 1.4 |
| Omnirad 380 | 0.250 | 0.5 |
| BHT | 0.050 | 0.1 |
| **Total** | **100.00** | **200** |

**Table 13: Compositions of Formulated Pastes based Combo Resins**

| **HEAVY BODY** | **AL02-171** | **Lot171212KR** |
|---|---|---|
| **ZZ1-208** | **%** | **Actual gms** |
| **Ratio of Resin A and Resin B** | **90/10** | |
| **Activated Combo Resin AL02-170-2** | 84.27 | 84.28 |
| Ken React LICA 09 | 0.13 | 0.13053 |
| Peppermint | 0.20 | 0.20633 |
| Aerosil 200 | 5 | 5 |
| Sipernat 50 | 10 | 10.05 |
| Corona Magenta Conc.(20%) | 0.4 | 0.40003 |
| **Total** | **100** | **100.06** |
| | | |
| **HEAVY BODY** | **AL02-172** | **Lot171212KR** |
| **ZZ2-4** | **%** | **Actual gms** |
| **Ratio of Resin A and Resin B** | **80/20** | |
| **Activated Combo Resin KR2-11** | 84.27 | 84.27 |
| Ken React LICA 09 | 0.13 | 0.13 |
| Peppermint | 0.20 | 0.20095 |
| Aerosil 200 | 5 | 5.02 |
| Sipernat 50 | 10 | 10.01 |

(continued)

| **HEAVY BODY** | **AL02-172** | **Lot171212KR** |
|---|---|---|
| **ZZ2-4** | **%** | **Actual gms** |
| **Ratio of Resin A and Resin B** | **80/20** | |
| Corona Magenta Conc.(20%) | 0.4 | 0.4094 |
| **Total** | **100** | **100.04** |

**Table 14: Properties of Formulated Light Curable Heavy Body Pastes based on Combo Resins**

| HB | TRON Resin Resin A 90% | Resin B 10% | Consistency mm @500g/30" | Strain Focus 8mm/20" top only Color 2min | Shore A@10mm 59mW/cm$^2$/30" (w/ dist. of 46mm) | DOC 59mW/cm$^2$/ 30" (w/ dist. of 46mm) | Tear Strength 100mm/min 30" in Triad top only | Compression Set (Recovery) |
|---|---|---|---|---|---|---|---|---|
| **AL02-132** | ZZ1-182 | ZZ1-181 | **31** | **19.55** | **42.2 (0.2)**<br>**51.0 (1.0)** | **15.7** | **319.2(6.9)** | **99.65** |
| **AL02-171** | AL02-170-2 w/ Combo Resin/ZZ1-208 | | **32.5** | **20.25** | **33.6 (1.3)**<br>**31.7 (1.1)** | **15.01** | **384.7(6.5)** | **99.35** |
| **AL02-172** | KR2-11 w/ Combo Resin/ZZ2-4 | | **34** | **20.65** | **21.1 (1.2)**<br>**19.9 (0.1)** | **11.06** | **300.3(15.2)** | **99.50** |

## Claims

1. A light-curable dental impression material comprising:

(a) a polymerizable polysiloxane resin composition comprising compounds of the following formula (I):

$$E\text{-}(L^1\text{-}Z)_n\text{-}L^2\text{-}E \qquad (I)$$

wherein

the E which may be the same or different, independently represent a monovalent group selected from a group containing a polymerizable carbon-carbon double bond, a group containing a polysiloxane moiety, a $C_{2-20}$ alkoxy group, a $C_{2-20}$ thioalkyl group, and a RNH group, wherein R is a $C_{2-20}$ alkyl group;
$L^1$ which may be the same or different when more than one $L^1$ is present, represents a divalent group of the following formula (II):

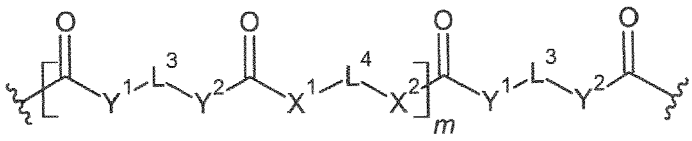

(II)

wherein

$L^3$ which may be the same or different when more than one $L^3$ is present, independently represents a divalent linker group which may be substituted with up to four substituents of the formula (III):

$$-L^1\text{-}E \qquad (III)$$

wherein $L^1$ and E are as defined above;
$L^4$ which may be the same or different when more than one $L^4$ is present, independently represents a divalent linker group which may be substituted with up to four substituents of the formula (III), wherein $L^1$ and E are as defined above;
$X^1$, $X^2$, $Y^1$, and $Y^2$, which may be the same or different, and when more than one $X^1$, $X^2$, $Y^1$, or $Y^2$, is present, the $X^1$, $X^2$, $Y^1$, and $Y^2$ may be the same or different, independently represent an oxygen atom, a sulfur atom and a group NR', wherein R' is a hydrogen atom or a $C_{1-4}$ alkyl group;
m represents 0 or an integer of from 1 to 40;

Z represents a divalent linker group which may additionally be substituted with up to four substituents selected from polysiloxane groups and groups of the formula (III), wherein $L^1$ and E are as defined above;
$L^2$ represents a single bond or a divalent group of the formula (II), wherein $L^3$, $L^4$, $X^1$, $X^2$, $Y^1$, $Y^2$ and m are independently as defined for $L^1$;
n represents 0 or an integer of from 1 to 4;

provided that

- a compound of formula (I) contains at least one monovalent group E having a polymerizable carbon-carbon double bond,
- a compound of formula (I) contains at least one polysiloxane group and

provided that
when n is 0, then $L^2$ is a divalent group of the formula (II);
(b) a particulate filler; and
(c) a photoinitiator.

2. The light-curable dental impression material according to claim 1, wherein the monovalent groups E contain

(meth)acrylate groups so that the total (meth)acrylate content of the polymerizable polysiloxane resin composition is in a range of from 0.20 to 0.50 mmol/g.

**3.** The light-curable dental impression material according to claim 1 or 2, wherein the polymerizable polysiloxane resin composition has a total siloxane content in a range of from 10 to 40% wt/wt.

**4.** The light-curable dental impression material according to any one of the preceding claims, wherein n is 0.

**5.** The light-curable dental impression material according to any one of the preceding claims, wherein $L^2$ represents a divalent group of the formula (II), wherein
$L^3$ is a divalent $C_{1-12}$ hydrocarbon group or a polysiloxane group;
$L^4$ which may be the same or different when more than one $L^4$ is present, independently represents a divalent linker group which may be substituted with up to four substituents of the formula (III), wherein $L^1$ and E are as defined above;
$X^1$ and $X^2$ are oxygen atoms,
$Y^1$ and $Y^2$ are NH groups, and
m is an integer of from 1 to 40.

**6.** The light-curable dental impression material according to any one of claims 1 to 3, wherein n>0.

**7.** The light-curable dental impression material according to any one of the preceding claims, wherein
$L^1$ and $L^2$ independently represent a divalent group of the formula (II), wherein
$L^3$ is a divalent $C_{1-12}$ hydrocarbon group or a polysiloxane group;
$L^4$ which may be the same or different when more than one $L^4$ is present, independently represents a divalent linker group which may be substituted with up to four substituents of the formula (III), wherein $L^1$ and E are as defined above;
$X^1$ and $X^2$ are oxygen atoms,
$Y^1$ and $Y^2$ are NH groups,
m is an integer of from 1 to 40; and
Z is a divalent $C_{1-12}$ hydrocarbon group.

**8.** The light-curable dental impression material according to any one of the preceding claims, wherein E represents a monovalent end-group of the following formula (IV):

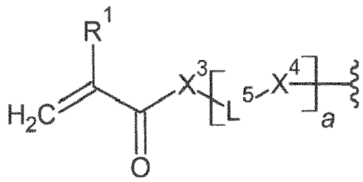

(IV)

wherein

$R^1$ represents a hydrogen atom or a $C_{1-12}$ alkyl group;
$X^3$ represents an oxygen atom, a sulfur atom or a group $NR^2$, wherein $R^2$ is a hydrogen atom, $C_{1-12}$ alkyl group, or an allyl group;
$L^5$ is a divalent hydrocarbon linker group or polysiloxane containing linker group, preferably a $C_{2-20}$ alkylene group; and
$X^4$ represents an oxygen atom, a sulfur atom or a group $NR^3$, wherein $R^3$ is a hydrogen atom, or a $C_{1-12}$ alkyl group; and
a represents an integer of from 1 to 20.

**9.** The light-curable dental impression material according to any one of the preceding claims, containing a polymerizable group selected from a (meth)acryloyl group, a (meth)acrylamide group or an allyl (meth)acrylamide group.

**10.** The light-curable dental impression material according to any one of the preceding claims, wherein E represents a polysiloxane group of the following formula (V):

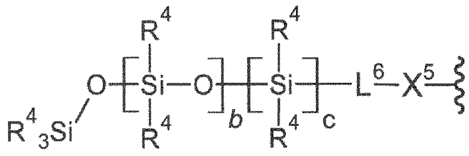

(V)

wherein

$R^4$ which may be the same or different, independently represent a straight-chain, branched or cyclic alkyl group;
$L^6$ is a divalent hydrocarbon linker group, preferably a $C_{2-20}$ alkylene group;
$X^5$ represents an oxygen atom, a sulfur atom or a group $NR^5$, wherein $R^5$ is a hydrogen atom, or a $C_{1-12}$ alkyl group;
b is 0 or an integer of from 1 to 1000; and
c is 0 or 1.

**11.** The dental material according to any one of the preceding claims, wherein $L^2$ and/or $L^3$ and/or $L^4$ independently represent a polysiloxane group of the following formula (VIII)

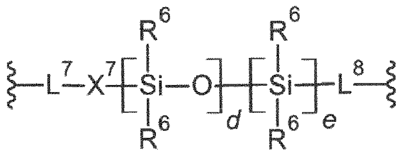

(VIII)

wherein

$R^6$ which may be the same or different, independently represent a straight-chain, branched or cyclic alkyl group, and a straight-chain, branched or cyclic alkoxy group;
$L^7$ and $L^8$ is a divalent hydrocarbon linker group, preferably a $C_{2-20}$ alkylene group;
$X^7$ represents an oxygen atom, a sulfur atom or a group $NR^7$, wherein $R^7$ is a hydrogen atom, or a $C_{1-12}$ alkyl group;
d is 0 or an integer of from 1 to 1000; and
e is 0 or 1, or

a polysiloxane group of the following formula (VIIIa), (VIIIb), or (VII(c)

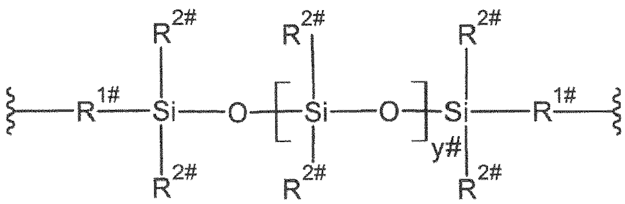

(VIIIa),

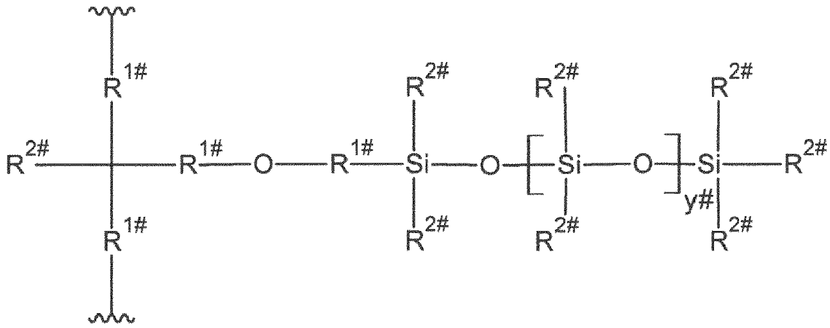

(VIIIb),

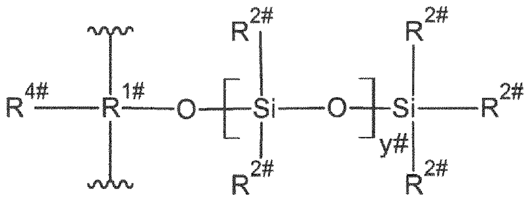

(VIIIc),

wherein

$R^{1\#}$ is an alkylene having 1 to 8 carbon atoms;
$R^{2\#}$ is an alkyl having from 1 to 4 carbon atoms;
$R^{4\#}$ is an alkyl, alkoxy or a cycloalkyl group;
$y^{\#}$ is an integer from 5 to 20.

**12.** The light-curable dental impression material according to any one of the preceding claims, wherein the compound of formula (I) has a molecular weight of 100 to 10.000 Da.

**13.** The light-curable dental impression material according to any one of the preceding claims, wherein $-L^1$-Z- represents a divalent group of the formula (II), which is obtainable by reacting a diisocyanate compound, a diol compound, and a polyol compound having at least three hydroxyl groups.

**14.** The light-curable dental impression material according to any one of the preceding claims, wherein $L^2$ represents a divalent group of the formula (II), which is obtainable by reacting a diisocyanate compound and a diol compound.

**15.** The light-curable dental impression material according to any one of the preceding claims, wherein the polymerizable polysiloxane resin mixture is obtainable by reacting a mixture comprising:

(a) x equivalents of one or more di- or polyol compounds of the following formula (Xs):

$$HO\text{-}L^4(OH)_l \qquad (X)$$

wherein

$L^4$ is an (l+1)-valent linker group; and
l is an integer of from 1 to 5;

(b) y equivalents of one or more compounds of the following formula (XI):

$$OCN\text{-}L^3NCO \qquad (XI)$$

wherein

$L^3$ is a divalent linker group; and
(c) z equivalents of one or more compounds of the following formula (XII):

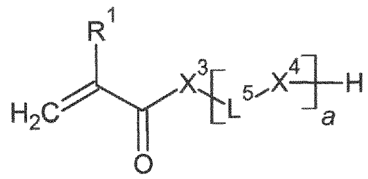

(XII)

wherein

$R^1$ represents a hydrogen atom or a $C_{1-12}$ alkyl group;
$X^3$ represents an oxygen atom, a sulfur atom or a group $NR^2$, wherein $R^2$ is a hydrogen atom, $C_{1-12}$ alkyl group, or an allyl group;
$L^5$ is a divalent hydrocarbon linker group, preferably a $C_{2-20}$ alkylene group; and
$X^4$ represents an oxygen atom, a sulfur atom or a group $NR^3$, wherein $R^3$ is a hydrogen atom, or a $C_{1-12}$ alkyl group,
a represents an integer of from 1 to 20.

wherein $0.05 \leq x/y \leq 0.66$, and $2y - \bar{f}x \leq z \leq 1.5(2y - \bar{f}x)$,
wherein x, y, and z are the molar equivalents of components (a), (b) and (c) and $\bar{f}$ is the mean hydroxyl functionality of component (a) : $\bar{f} = \sum_{l=1}^{3} \frac{x_l}{x}(l+1)$ wherein l is as defined in formula (X) and xi/x is the molar fraction of the compounds having a hydroxyl functionality of l+1.

**16.** The light-curable dental impression material according to any one of the preceding claims, which comprises

(i) 2 to 95 percent by weight, based on the total weight of the light-curable dental impression material, of a polymerizable polysiloxane resin composition comprising compounds of formula (I), and which comprises
(ii) 5 to 50 percent by weight, based on the total weight of the light-curable dental impression material, of a particulate filler; and
(iii) 0.1 to 5 percent by weight, based on the total weight of the light-curable dental impression material, of a photoinitiator.

**17.** The light-curable dental impression material according to any one of the preceding claims, which additionally comprises a compound of formula (I) which does not contain any polysiloxane group.

**18.** Use of a light-curable dental impression material according to any one of the preceding claims for the preparation of a dental impression.

Fig. 1

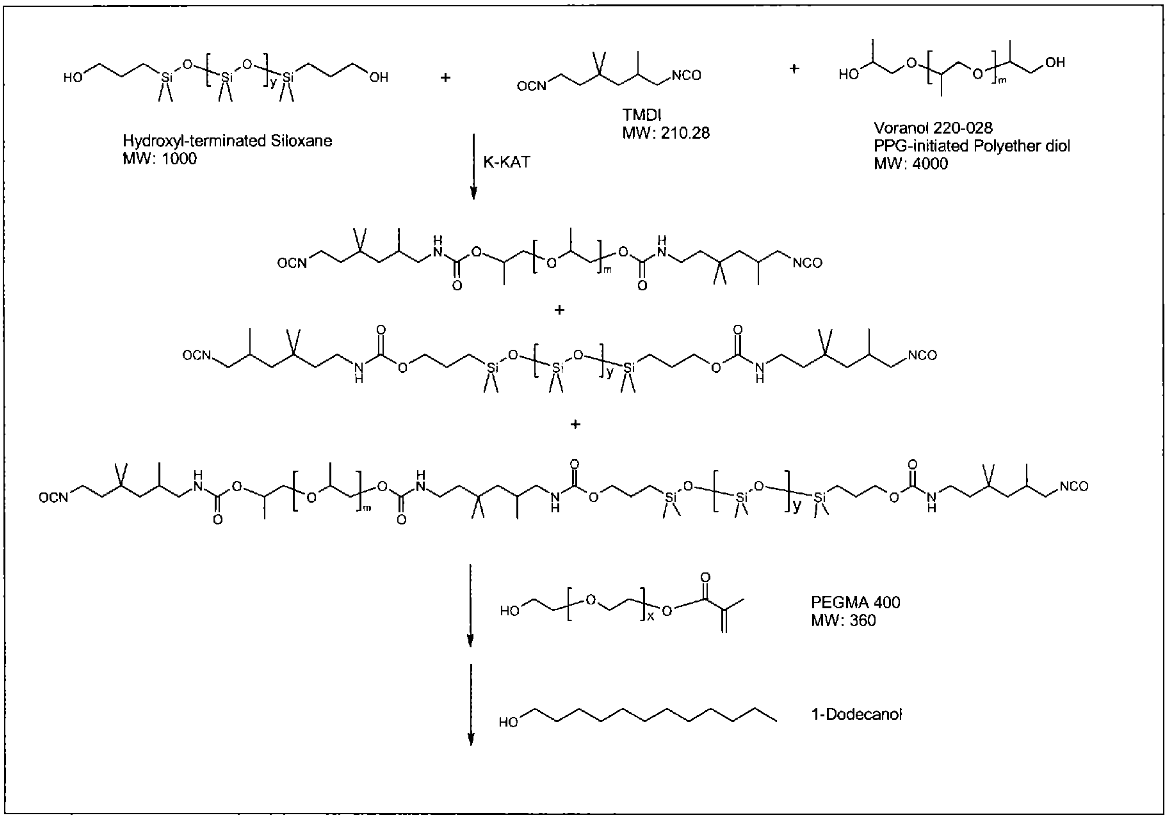
Hydroxyl-terminated Siloxane
MW: 1000
TMDI
MW: 210.28
Voranol 220-028
PPG-initiated Polyether diol
MW: 4000
K-KAT
PEGMA 400
MW: 360
1-Dodecanol

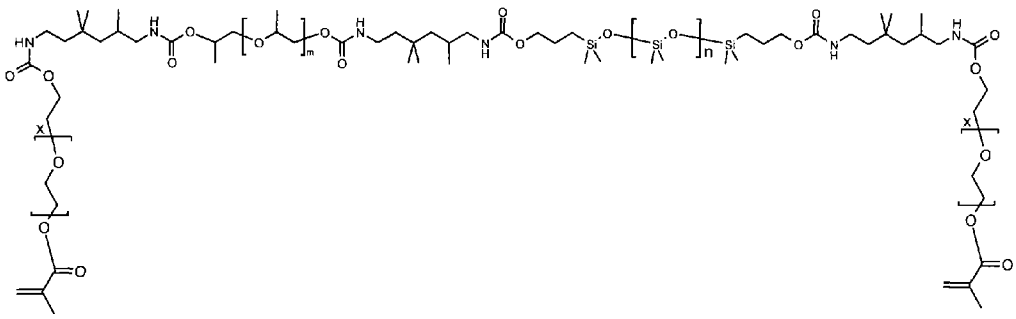

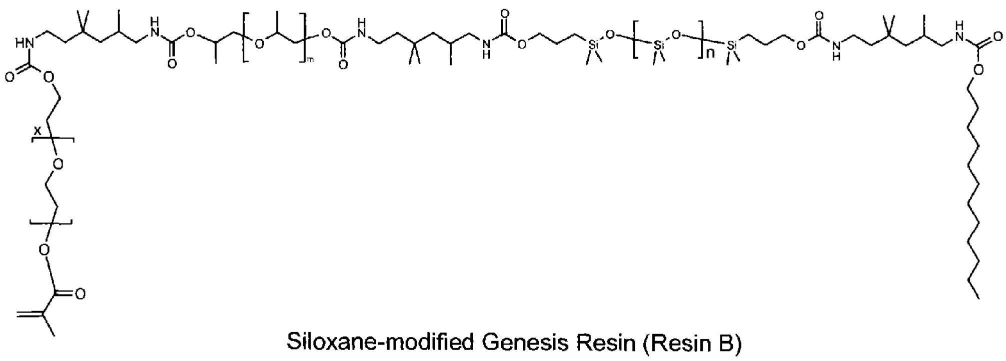

Siloxane-modified Genesis Resin (Resin B)

Fig. 2

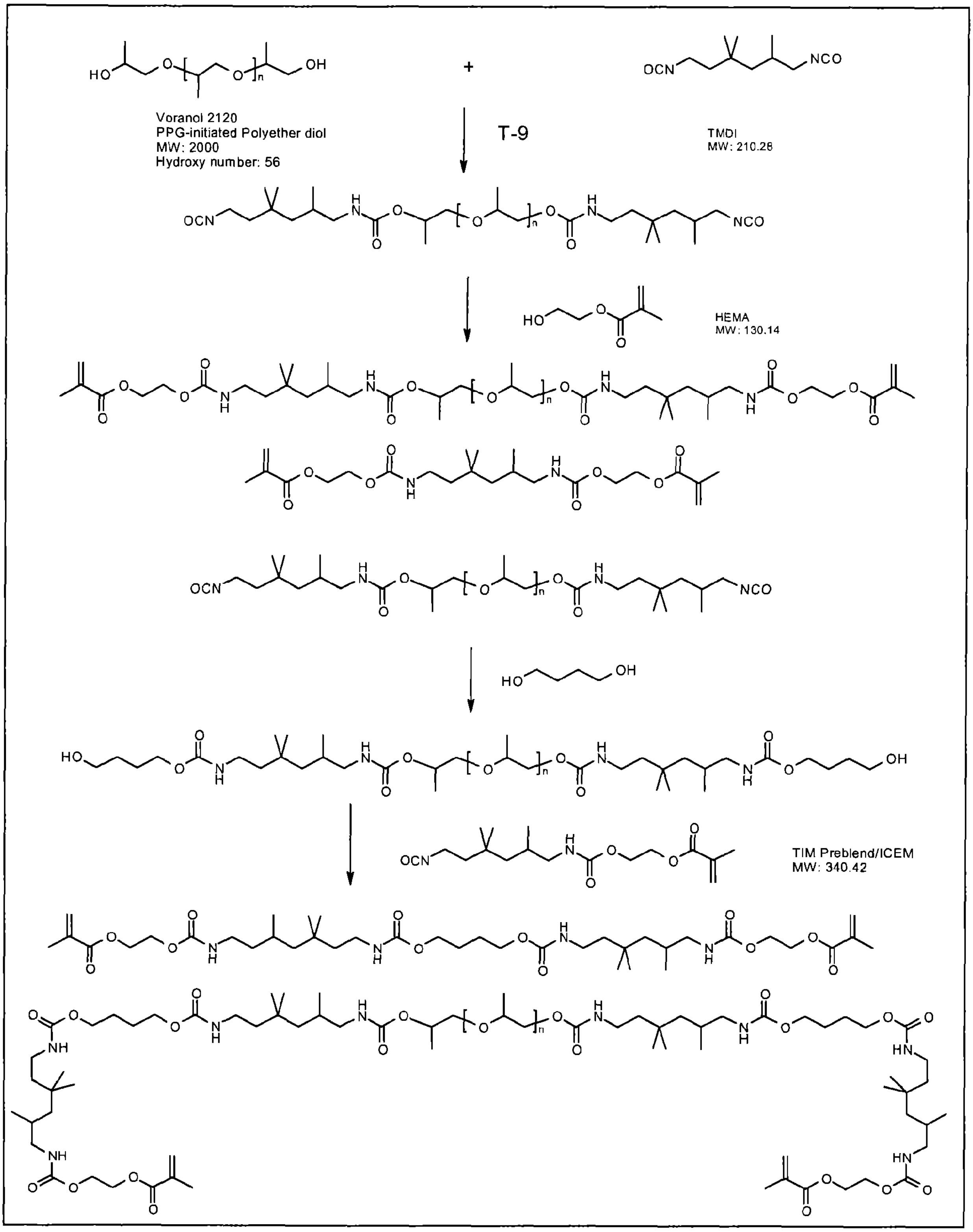
Voranol 2120
PPG-initiated Polyether diol
MW: 2000
Hydroxy number: 56
T-9
TMDI
MW: 210.26
HEMA
MW: 130.14
TIM Preblend/ICEM
MW: 340.42

Fig. 3

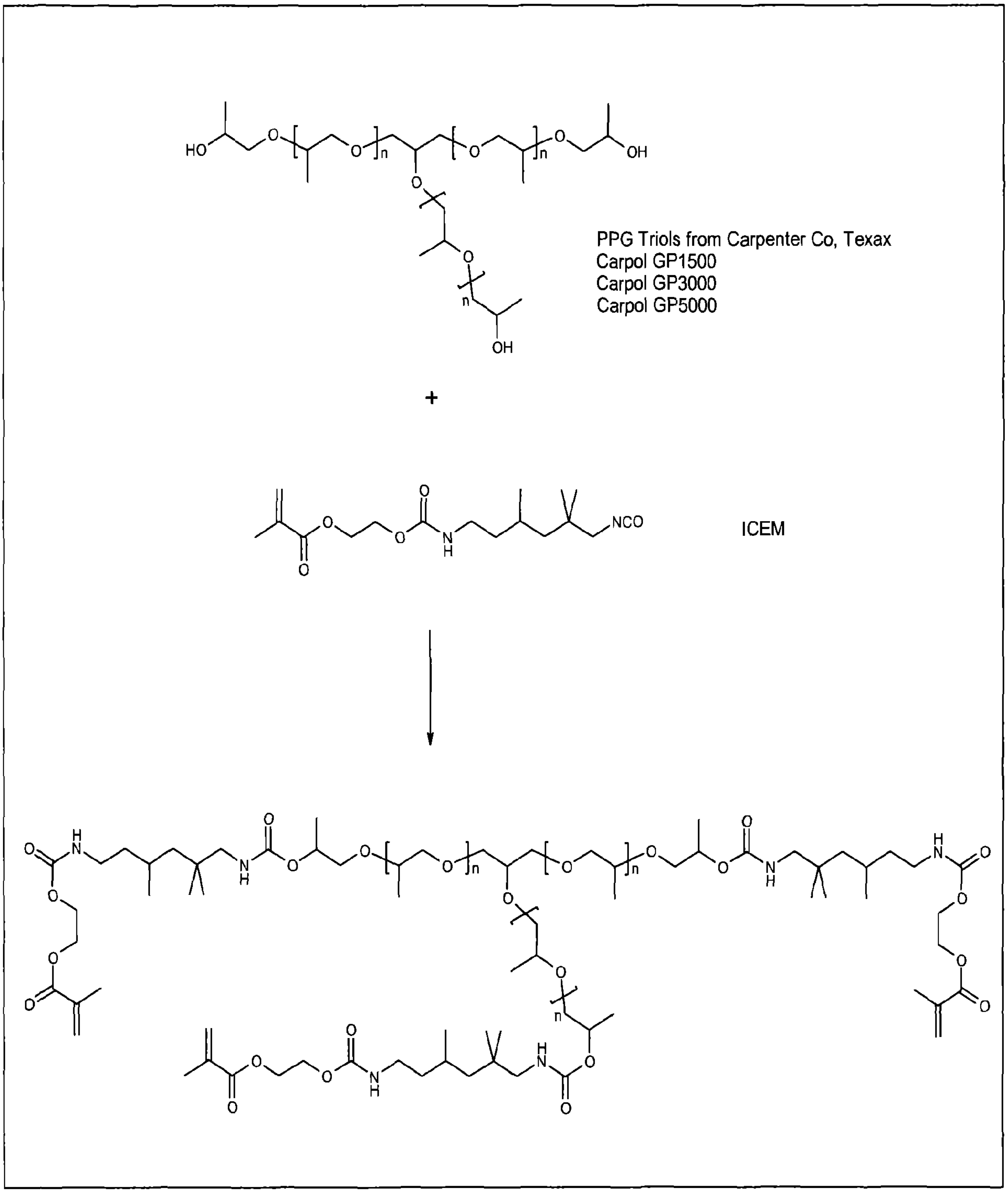
PPG Triols from Carpenter Co, Texax
Carpol GP1500
Carpol GP3000
Carpol GP5000
ICEM

Fig. 4

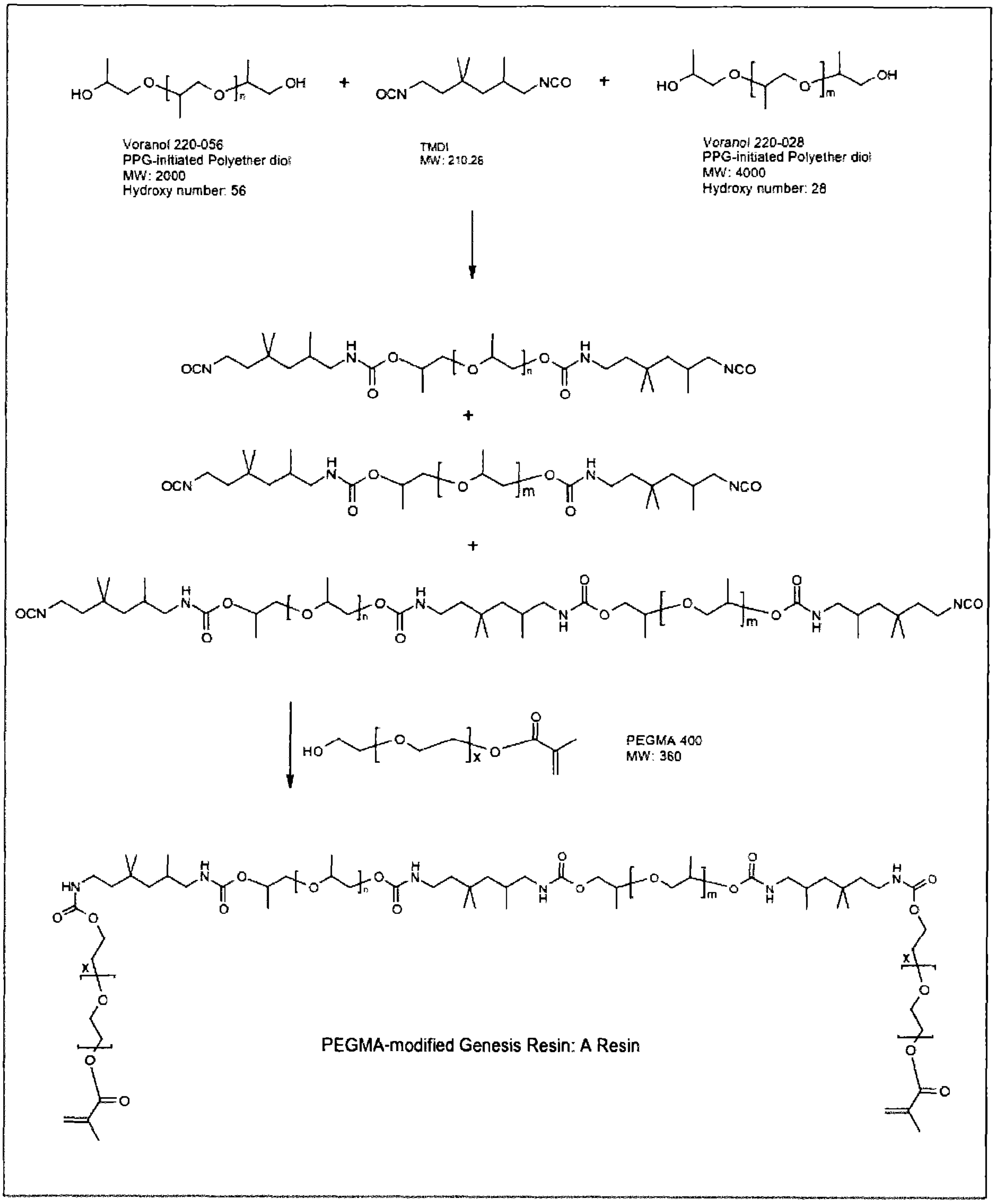
Voranol 220-056
PPG-initiated Polyether diol
MW: 2000
Hydroxy number: 56
TMDI
MW: 210.26
Voranol 220-028
PPG-initiated Polyether diol
MW: 4000
Hydroxy number: 28
PEGMA 400
MW: 360
PEGMA-modified Genesis Resin: A Resin

Europäisches Patentamt
European Patent Office
Office européen des brevets

# EUROPEAN SEARCH REPORT

Application Number
EP 18 19 8048

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JIYE CHENG ET AL: "Synthesis and properties of photopolymerizable bifunctional polyether-modified polysiloxane polyurethane acrylate prepolymer", JOURNAL OF ADHESION SCIENCE AND TECHNOLOGY, vol. 30, no. 1, 2016, pages 2-12, XP002791112, DOI: 10.1080/01694243.2015.1087255 * abstract * * page 10 * * page 5, paragraph 2.4 * ----- | 1-9, 11-16 | INV. A61K6/10 |
| A,D | US 5 137 448 A (DOUGHERTY EMERY W [US] ET AL) 11 August 1992 (1992-08-11) * claims 1-13 * ----- | 1-18 | |
| A,D | US 5 849 812 A (ZECH JOACHIM [DE] ET AL) 15 December 1998 (1998-12-15) ----- | 1-18 | TECHNICAL FIELDS SEARCHED (IPC) |
| A,D | EP 3 231 413 A1 (DENTSPLY DETREY GMBH [DE]) 18 October 2017 (2017-10-18) * claims 1-15 * ----- | 1-18 | A61K |
| A,D | US 5 545 676 A (PALAZZOTTO MICHAEL C [US] ET AL) 13 August 1996 (1996-08-13) * claims 1-35 * ----- | 1-18 | |

The present search report has been drawn up for all claims

2

| Place of search | Date of completion of the search | Examiner |
|---|---|---|
| Berlin | 9 May 2019 | Siatou, Evangelia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 18 19 8048

This annex lists the patent family members relating to the patent documents cited in the above-mentioned European search report. The members are as contained in the European Patent Office EDP file on The European Patent Office is in no way liable for these particulars which are merely given for the purpose of information.

09-05-2019

| Patent document cited in search report | Publication date | Patent family member(s) | Publication date |
|---|---|---|---|
| US 5137448 A | 11-08-1992 | US 5137448 A | 11-08-1992 |
| | | US 5387105 A | 07-02-1995 |
| US 5849812 A | 15-12-1998 | DE 19719438 A1 | 13-11-1997 |
| | | US 5849812 A | 15-12-1998 |
| EP 3231413 A1 | 18-10-2017 | AU 2017249668 A1 | 19-07-2018 |
| | | CA 3011777 A1 | 19-10-2017 |
| | | CN 108697587 A | 23-10-2018 |
| | | EP 3231413 A1 | 18-10-2017 |
| | | JP 2019510747 A | 18-04-2019 |
| | | US 2019117522 A1 | 25-04-2019 |
| | | WO 2017178383 A1 | 19-10-2017 |
| US 5545676 A | 13-08-1996 | BR 8801531 A | 08-11-1988 |
| | | CA 1323949 C | 02-11-1993 |
| | | DE 3856261 D1 | 03-12-1998 |
| | | DE 3856261 T2 | 17-06-1999 |
| | | EP 0290133 A2 | 09-11-1988 |
| | | JP 2744789 B2 | 28-04-1998 |
| | | JP S63273602 A | 10-11-1988 |
| | | US 5545676 A | 13-08-1996 |
| | | US 6017660 A | 25-01-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5849812 A
- US 5137448 A
- US 5545676 A
- EP 3231413 A1
- EP 3153150 A1
- US 4298738 A
- US 4324744 A
- US 4385109 A
- EP 0173567 A


### Non-patent literature cited in the description


- *CHEMICAL ABSTRACTS,* 1565-94-2
- *CHEMICAL ABSTRACTS,* 72869-86-4